# EUROPÄISCHE PATENTANMELDUNG

(11) **EP 0 771 795 A1**
(43) Veröffentlichungstag der Anmeldung: **07.05.1997**
(21) Anmeldenummer: 96116670.9
(22) Anmeldetag: 17.10.1996
(51) Int. Cl.: C07D 309/10, C07D 307/20, C07D 405/10, C07D 407/06, A61K 31/35

(54) **Neuartige Glycomimetika als Selektin-Antagonisten und entzündungshemmend wirkende Arzneimittel**

(30) Priorität: 30.10.1995 DE 19540388
(71) Anmelder: HOECHST AKTIENGESELLSCHAFT, 65929 Frankfurt am Main (DE)
(72) Erfinder: Schmidt, Wolfgang, Dr., 65929 Frankfurt (DE); Sprengard, Ulrich, 65462 Gustavsburg (DE); Kretzschmar, Gerhard, Dr., 65760 Eschborn (DE); Klein, Robert, Dr., 65933 Frankfurt (DE); Kunz, Horst, Prof. Dr., 55127 Mainz (DE)

(57) **Zusammenfassung**

Die Erfindung betrifft neue Mimetika der Tetrasaccharide Sialyl Lewis-X und Sialyl Lewis-A mit verbesserter Wirkung als Inhibitoren der Zelladhäsion, ein Verfahren zur Herstellung dieser Verbindungen sowie deren Verwendung als pharmakologische Wirkstoffe und Diagnostika.

## Beschreibung

Die Erfindung betrifft neue Mimetika der Tetrasaccharide Sialyl Lewis-X (SLeX) und Sialyl Lewis-A (SLeA) mit verbesserter Wirkung als Inhibitoren der Zelladhäsion, ein Verfahren zur Herstellung dieser Verbindungen sowie deren Verwendung als pharmakologische Wirkstoffe und Diagnostika.

Die Zirkulation von Blutzellen wie z.B Leukozyten, Neutrophilen, Granulozyten und Monozyten ist auf molekularer Ebene ein vielstufiger, sehr komplexer Prozess, der nur in Teilschritten bekannt ist (Review: T.A.Springer, Cell 76, 301-314, 1994).
Jüngste Forschungsergebnisse zeigten, daß die in der Immunüberwachung entscheidende Rezirkulation der Lymphozyten sowie die Lokalisierung von Neutrophilen und Monozyten an Entzündungsherden sehr ähnlichen molekularen Mechanismen gehorchen. So kommt es bei akuten und chronischen Entzündungsprozessen zur Adhäsion der Leukozyten an Endothelzellen und Auswanderung in den Entzündungsherd und in die sekundären lymphatischen Organe.
An diesem Vorgang sind zahlreiche spezifische Signalmoleküle wie z.B. Interleukine, Leukotriene und Tumornekrosefaktor (TNF), deren G-Protein gekoppelte Rezeptoren und insbesondere gewebespezifische Zelladhäsionsmoleküle beteiligt, die eine genau kontrollierte Erkennung der Immun- und Endothelzellen gewährleisten. Zu den wichtigsten hierbei beteiligten Adhäsionsmolekülen, die im folgenden als Rezeptoren bezeichnet werden sollen, gehören die Selektine (E-, P- und L-Selektine), Integrine und die Mitglieder der Immunglobulin-Superfamilie.

Die in der Anfangsphase entzündlicher Prozesse durch Selektin-Rezeptoren vermittelte Adhäsion von Leukozyten an Endothelzellen ist eine natürliche und notwendige Immunantwort auf diverse Entzündungsreize und Verletzungen des vasculären Gewebes.
Der Verlauf einer Reihe von akuten und chronischen Erkrankungen wie beispielsweise Rheuma, Reperfusionsverletzungen wie myokardiale Ischämie/Infarkt (MI), akute Lungenentzündung nach operativem Eingriff, traumatischer Schock und Schlaganfall, Psoriasis, Dermatitis, ARDS (Atemnotsyndrom bei Erwachsenen) sowie die nach chirurgischen Eingriffen (Beispiel Angioplastie und By-Pass Operationen) auftretende Restenose wird jedoch durch die übermäßige Adhäsion von Leukozyten und deren Infiltration in das betroffene Gewebe ungünstig beeinflußt. Die Beeinflussung dieses Adhäsionsprozesses in einem sehr frühen Stadium der Entzündung ist deshalb ein sehr attraktives und generell anwendbares Konzept zur pharmakologischen Kontrolle entzündlicher Erkrankungen.

Es wird heute generell anerkannt, daß die Tetrasaccharide Sialyl Lewis-X (SLeX) und Sialyl Lewis-A (SLeA), die als Teilstrukturen von Glycosphingolipiden und Glycoproteinen auf Zellmembranen vorkommen, als Liganden für alle drei Selektinrezeptoren fungieren können.
In der Literatur werden eine Reihe von Glycoproteinen, Mucinen und Glycolipide beschrieben, die als endogene Liganden der Selektine in Frage kommen. Dazu gehören das Mucosal Vascular Addressin MadCAM-1 (Berg et al., Nature 1993, 366, 695) und das Sialomucin CD34 (Baumhuter et.al., Science 1993, 262, 436) für L-Selektin, ein O-linked Polylactosamin-Sialomucin PSGL-1 auf humanen Neutrophilen für P-Selektin (Moore et.al., J.Biol.Chem. 1994, 269, 23318) und N-linked Sialoglycoproteine vom Typ ESL-1 für E-Selektin (Vestweber et.al., Cell Biol. 1993, 121, 449).

Die Spezifität dieser und anderer potentieller Liganden für Selektine in vivo ist noch nicht geklärt. Die auf Selektin-Liganden vorkommenden Tetrasaccharide SLeX und SLeA repräsentieren lediglich eine Teilstruktur der wesentlich komplexeren endogenen Ligandenstrukturen und können aufgrund ihrer sehr ähnlichen Affinität zu Selektinen nicht allein zur Erklärung einer spezifischen Rezeptorbindung herangezogen werden.
Aufgrund der Komplexität dieser Strukturen ist der Versuch, die an Selektine bindende Tetrasaccharide SLeX/A als Teilstrukturen in verschiedenen Darreichungsformen oder einfache Mimetika derselben mit modifizierter Struktur als Antagonisten zur Modulierung oder Unterbindung einer übermäßigen Leukozytenadhäsion einzusetzen und so zur Linderung bzw. Heilung genannter Erkrankungen beizutragen, ein vielversprechender therapeutischer Ansatz.

Der natürliche Ligand mit der Struktur von SLeX wurde schon erfolgreich in Tierversuchen bei P-Selektin abhängigen Lungenverletzungen (M.S.Mulligan et.al., Nature 1993, 364, 149) und bei myokardialen Reperfusionsverletzungen (M.Buerke et.al., J.Clin.Invest. 1994, 93, 1140) verwendet. In ersten klinischen Prüfungen bei akuter Lungenentzündung soll die Verbindung in einer Dosis von 1-2 Gramm pro Tag und Patient eingesetzt werden (Mitteilung der Firma Cytel Corp,/ La Jolla (CA.) beim 2. Glycotechnology Meeting/CHI in La Jolla/USA am 16.-18. Mai 1994).

In einigen Publikationen und Patentanmeldungen wurde inzwischen über Bemühungen berichtet, durch strukturelle Variation des Liganden zu fester bindenden Antagonisten zu Bemühungen ist gelangen. Ziel dieser Arbeiten ist die Bereitstellung wirksamerer Antagonisten, die auch potentiell in vivo bei geringerer Dosis einsetzbar wären.

Die Variation der für die Struktur-Wirkungs-Beziehung bislang als entscheidend angesehenen Fucose- und Neuraminsäurebausteine (B.K.Brandley et.al., Glycobiology 1993, 3, 633 und M.Yoshida et.al., Glycoconjugate J. 1993, 10, 3) erbrachte jedoch keine signifikant verbesserten Inhibitionswerte. Allein bei Variation des Glucosaminbausteins (Ersatz von GlcNAc durch Glucose und Azido- sowie Aminogruppen in der 2-Position von GlcNAc) ließ sich eine signifikant erhöhte Affinität an den E-Selektin-Rezeptor erreichen. Beim P-Selektin-Rezeptor wurde hingegen keine verbesserte Bindung erreicht.

Generell blieben bisher alle Erfolge zur Verbesserung der Bindungsaffinität von SLeX-und SLeA-Derivaten auf den E-Selektinrezeptor beschränkt, denn mit dem P-Selektinrezeptor wurden nur schwache Inhibitionseffekte bei Inhibitorkonzentrationen von ca. 1 mM gefunden (R.M.Nelson et.al., J.Clin.Invest. 1993, 91, 1157).
Der Stand der Technik zur Bindungsaffinität modifizierter SLeX/A-Strukturen an Selektine wird in Pharmacochem. Libr. 1993, 20 (Trends in Drug Research), Seiten 33-40 referiert.

Neben der geringen Bindungsaffinität an die Selektine, die diese Verbindungen aufweisen, enthalten sie jedoch alle mindestens eine labile glycosidische Bindung, was die orale Verfügbarkeit dieser Wirkstoffe extrem einschränkt. Diese Labilität schränkt auch den Aufbau von verschiedenen Derivaten stark ein, da die empfindliche, zur Spaltung neigende glycosidische Bindung die Reaktionsbedingungen stark einschränkt. Verschiedenste Ansätze der Synthese von Mimetika wurden bearbeitet, um zu einer Erhöhung der Stabilität zu gelangen
Eine Erhöhung der Stabilität erfolgte durch die Anknüpfung der Seitenkette über eine C-C Verknüpfung an dem Kohlenstoff C-4 der Fucose (Floyd et al, Tetrahedron Asymmetry 1994, 5, 2061).

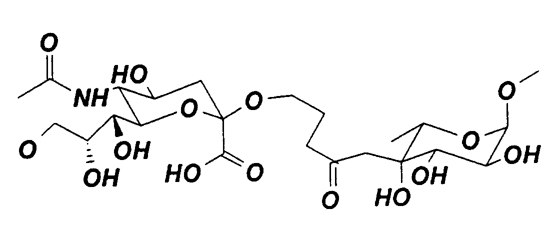

Hierbei weicht jedoch durch die Anknüpfung an C-4 der Fucose die Orientierung der Seitenkette Von der des natürlichen Liganden ab. Es zeigte sich nur eine sehr geringe Affinität zur Bindung an die Selektine.

Der Einsatz carbacyclischer Kohlenhydratanaloga, bei der die Anknüpfung der Seitenkette durch eine C-C Bindung an C-1 erfolgt, würde eine dem natürlichen Liganden ähnliche Konformation aufweisen, aber trotzdem gegen Abbau stabil sein. Mit großer Intensität wird an der Darstellung carbacyclischer Kohlenhydratanaloga von Monosaccharidbausteinen gearbeitet.
Von den verschiedenen Methoden sind die Umsetzungen von aktivierten Monosacchariden mit Nitromethan (Gross P.H., Tetrahedron 1991, 47,6113), Allylsilan (Y. Kishi et. al., J. Am. Chem. Soc. 104, 4976-4978, 1982) und Olefinen (D.A. Levy et. al., Tetrahedron Asymmetry 5, 2265-2268, 1994) hervorzuheben, die sich aufgrund der eingeführten Funktionalität als Baustein für weitere Kupplungen eignen.
Die Nutzung eines carbacyclischen Analogons als Baustein für Selektinantagonisten führte zu einem Mimetikum mit Affinität an Selektine. Hierbei konnte durch Umsetzung eines Fucosebausteines mit Allylsilan ein spezieller C-glycosidischer Baustein 1 mit α-Orientierung der Seitenkette aufgebaut werden (WO 95/04751). Die Selektivitäten in der Allylierung sind mit α/β = 14/1 sehr gut, jedoch ist ein up-scaling der Reaktion aufgrund der Bedingungen (10 Equivalentel Trimethylsilyltriflat erforderlich) nicht einfach möglich. Ebenfalls ist eine chromatographische Reinigung des Bausteines erforderlich, bei der sich das α/β Gemisch jedoch nicht trennen läßt.

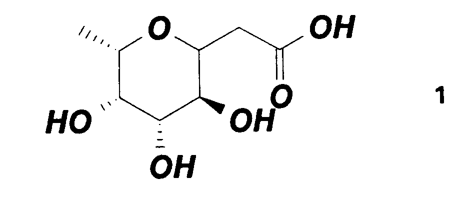

Die endständige Säurefunktion der Seitenkette wird zum Aufbau von Glycopeptidanaloga (z.B. **2**) eingesetzt. Die Derivate weisen IC₅₀-Werte im Bereich von bis zu 1 mM auf. Die Glycopeptidanaloga sind jedoch gegen den Abbau der Peptidkette durch Proteasen nicht stabil, so daß die orale Verfügbarkeit dieser Verbindungen, obwohl der Zuckerbaustein durch das C-Glycosid stabil geworden ist, weiterhin stark eingeschränkt ist. Ein weiterer Nachteil ist die β-Verbindung, die bei dieser Synthese nicht abgetrennt werden kann. Die entsprechenden β-Derivate weisen keine Wirksamkeit auf, da die Seitenkette falsch orientiert wird, wie entsprechende Modellrechnungen belegen.

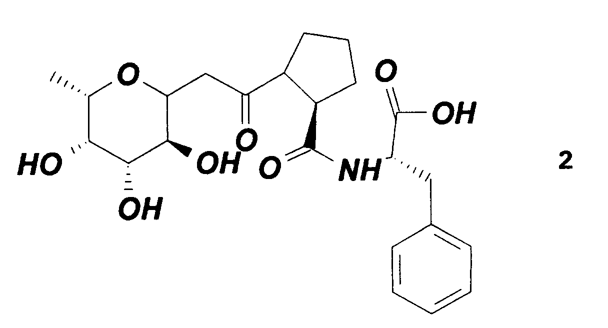

In analoger Weise wurde der C-glycosidische Baustein 1 ebenfalls zum Aufbau verschiedener weiterer Mimetika eingesetzt, die die aktive Konformation des SLe^{x} imitieren sollten. Die getesteten Verbindungen z.B. **3** zeigten jedoch mit 10-20 mM um den Faktor 10-20 höhere IC₅₀-Werte als der natürliche Ligand SLe^{x} (Wong, et. al., J. Am. Chem. Soc. 1995, 117, 5395).

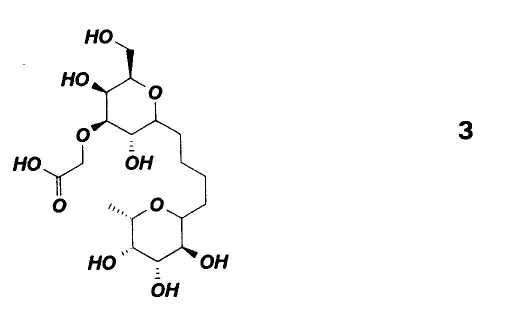

Durch den Einsatz eines substituierten Allylsilans konnte das C-Glycosid dargestellt werden.

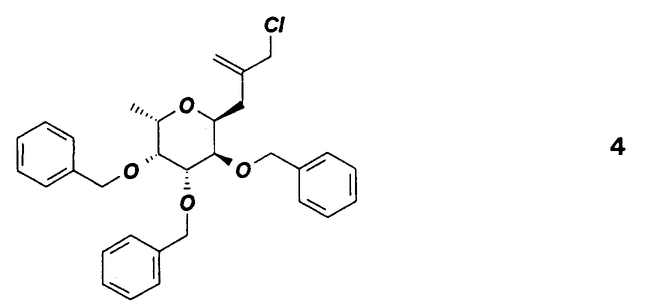

Durch Anknüpfung an Triterpenoid Säurederivate (Betulinic Acid: WO 95/04526; Glycyrrhetinic Acid: WO 94/24145) 4a wurden Mimetika dargestellt, die eine "multi-medicament capacity" aufweisen sollen und in verschiedensten Testsystemen getestet wurden (Inhibierung von 5-Lipoxygenase, Antimetastatische Wirkung, P-Selektin Hemmung). Hierbei ergab sich für die Bindung an P-Selektin ein IC₅₀-Wert von 0.75 mM. Zu beachten ist hierbei jedoch, das allein die Triterpenoid Säure in diesem Test schon Werte von 0.125 mM aufweist.

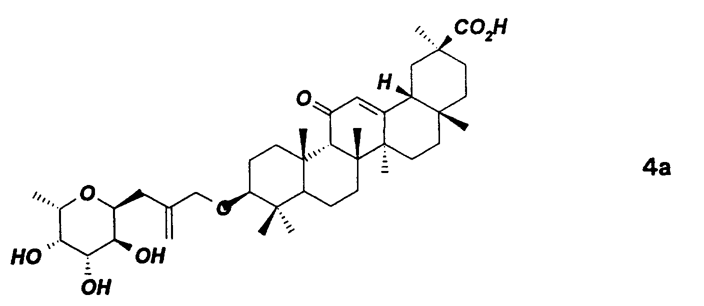

Die Darstellung des C-Glycosidbausteines 4 erfordert ebenfalls eine aufwendige chromatographische Reinigung, bei der das β-Derivat nur schwer abgetrennt werden kann. Auch die Stabilität des C-Glycosids ist durch die reaktive Allylchlorid-Gruppierung eingeschränkt.

Die genannten C-Glycosid Mimetika weisen neben den bereits beschriebenen präparativen Problemen (aufwendige chromatographische Reinigung, hohe Stufenzahl durch Schutzgruppenstrategie, α/β Gemisch) eine zu geringe Bindungsaffinität an die Selektine auf, um in die Adhäsionsprozesse wirksam eingreifen zu können. Denn neben dem Aspekt der reinen Stabilität eines Fucose-Mimetikums ist für die Bindung an die Selektine auch die Ausrichtung und konformative Fixierung der Seitenkette von besonderer Bedeutung, die die genannten Derivate anscheinend nicht erfüllen.

Nach L.A. Lasky ist die negativ geladene Sialinsäure (oder eine negativ geladenen Sulfonsäuregruppe) absolut notwendig für die Bindung an Selektine. Da die Kristallstruktur von E-Selektin aufgeklärt werden konnte, wurden bereits Untersuchungen über mögliche Bindungsstellen durchgeführt. Als mögliche Bindungstellen für die Sialinsäurefunktion wurden hierbei zum einen die beiden Lysine K111 bzw. K113 (J.Bajorath et.al., Biochemistry 1994, 33, 1332) und zum anderen Arg 97, Lys 111 und Lys 113 (Structural Biology 1, 140 (1994)) vorgeschlagen.

Demgegenüber ist es Aufgabe der vorliegenden Erfindung, stabile, niedermolekulare Mimetika der Sialyl-Lewis-X bzw. Sialyl-Lewis-A-Strukturen bereitzustellen, deren Konstitution und Konfiguration eine signifikant gesteigerte Affinität an Selektine besitzen, synthetisch einfacher als Oligosaccharide zugänglich sind und strukturbedingt als potentiell oral verfügbare Arzneimittel geeignet sind.

Die gestellte Aufgabe wird gelöst durch
1. eine Verbindung der Formel I 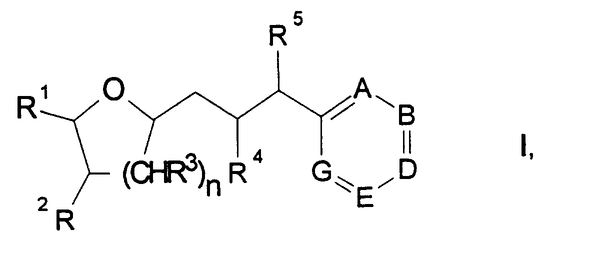 worin bedeuten
   - n: 1 oder 2,
   - R¹: -H, -CH₂OH oder -CH₃,
   - R² und R³: unabhängig voneinander -H oder -OH,
   - R⁴ und R⁵: unabhängig voneinander -H, -OH, -Alkyl, -O-Alkyl, -S-Alkyl, -NH₂, -NH-Alkyl, -N(Alkyl)₂, -NH-Aryl, -N(Aryl)₂, -OSO₃H, -(CH₂)r-COOH, -(CH₂)ᵣ-COO-Alkyl, -(CH₂)ᵣCH(COO-Alkyl)₂, -(CH₂)ᵣCH(COOH)₂, -(CH₂)ᵣ-NH₂, wobei r eine ganze Zahl von null bis zehn bedeutet, oder gemeinsam eine Doppelbindung oder einen Epoxidring,
   - A, B, D, E und G: CR⁶, CR⁷, CR⁸, CR⁹, CR¹⁰ oder Stickstoff mit der Maßgabe, daß jeweils nur eine der Variablen A, B, D, E oder G Stickstoff bedeutet, und
   - R⁶, R⁷, R⁸, R⁹ und R¹⁰: unabhängig voneinander -H, -Alkyl, -OH, -O-Alkyl, -NH₂, -NH-Alkyl, -N(Alkyl)₂, -NH-Aryl, -N(Aryl)₂, -F, -Cl, -Br, -I, -COO-Alkyl, -CO-NH₂, -COOH, -OSO₃H, 4-Hydroxy-piperidin-4-yl, -(CH₂)ₘ-COOH, -(CH₂)ₘCOO-Alkyl, -(CH₂)ₘ-CH(COO-Alkyl)₂, -(CH₂)ₘ-CH(COOH)₂, wobei m eine ganze Zahl von null bis zehn bedeutet,
   -(CH₂)ₚ-NH₂, wobei p eine ganze Zahl von eins bis zehn bedeutet, eine Gruppe der Formel II 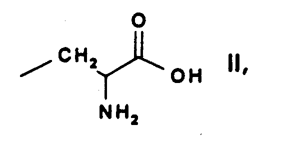 eine Gruppe der Formel III 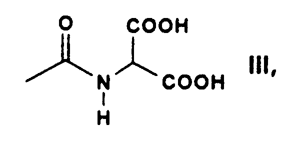 eine Gruppe der Formel IV 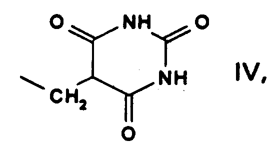 eine Gruppe der Formel V 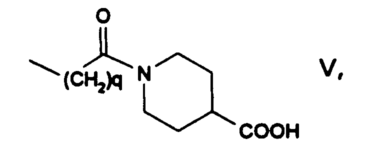 wobei q eine ganze Zahl von eins bis zehn bedeutet,
   oder eine Gruppe der Formel VI 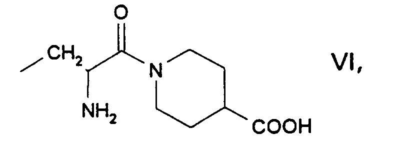 oder eine Gruppe der Formel VII 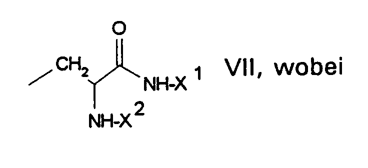 X¹ und X² unabhängig voneinander H oder ein Oligopeptid darstellen oder
   zwei der Variablen R⁶, R⁷, R⁸,R⁹ oder R¹⁰ bilden, sofern diese benachbart sind, gemeinsam einen mit einer Carboxymethylgruppe substituierten Imidazolring oder einen Kronenetherring, wobei die übrigen Variablen die genannte Bedeutung haben.

   Vorzugsweise ist die Verbindung der Formel I dadurch gekennzeichnet, daß
2. eine der Variablen A,B, G, E oder D C-COOH und alle übrigen dieser Variablen C-H bedeuten, beispielsweise 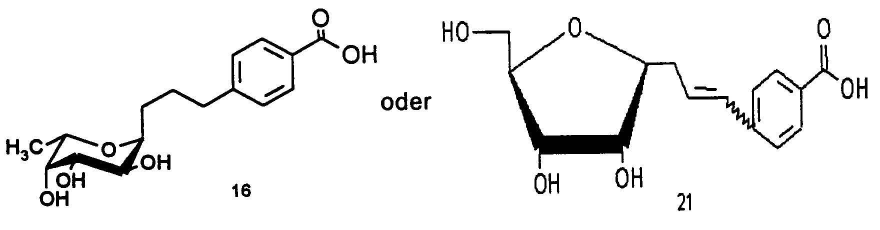 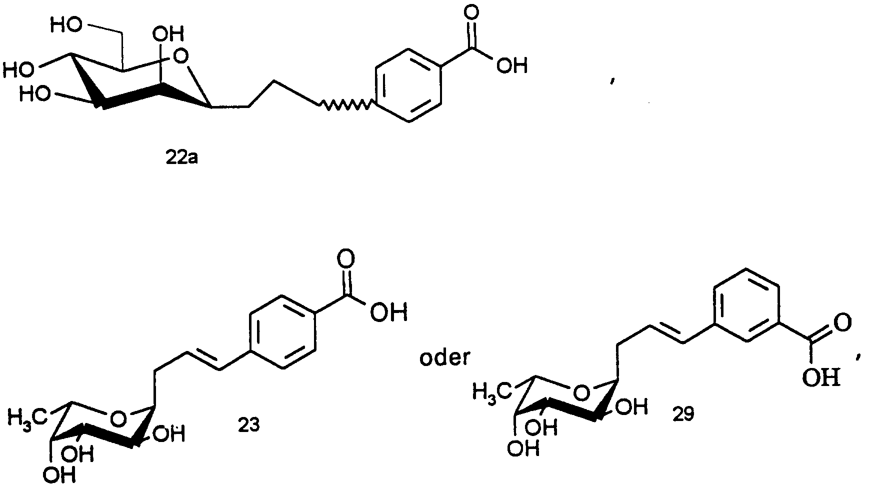
3. oder dadurch gekennzeichnet, daß eine der Variablen A,B, G, E oder D C-CH₂-COOH und alle übrigen dieser Variablen C-H bedeuten, beispielsweise 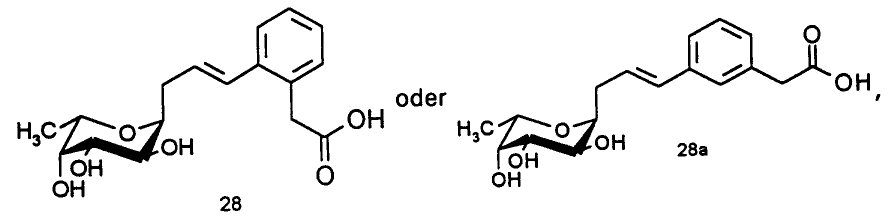
4. oder dadurch gekennzeichnet, daß eine der Variablen A,B, G, E oder D C-CH(COOH)₂ und alle übrigen dieser Variablen C-H bedeuten, beispielsweise 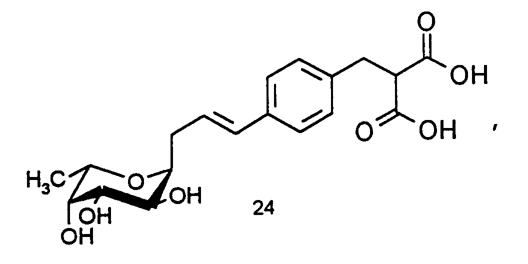
5. oder dadurch gekennzeichnet, daß eine der Variablen A,B, G, E oder D C-NH₂ und alle übrigen dieser Variablen C-H bedeuten, beispielsweise 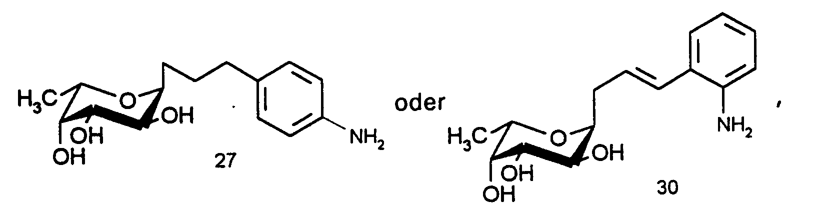
6. oder dadurch gekennzeichnet, daß die Variablen
   A, D und G C-H, die Variable B Stickstoff und die Variable E C-CH₂-COOH bedeuten, beispielsweise 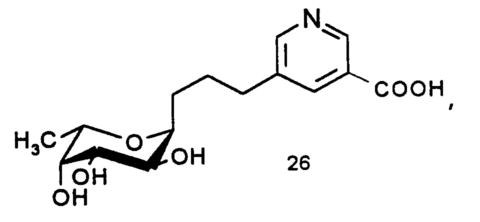
7. oder dadurch gekennzeichnet, daß
   - A,B, G und E: C-H und
   - D: CR⁸ bedeuten,
8. besonders bevorzugt dadurch gekennzeichnet, daß
   - R⁸: eine Gruppe der Formel II 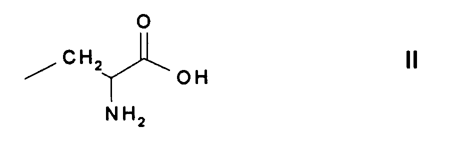 bedeutet, beispielsweise 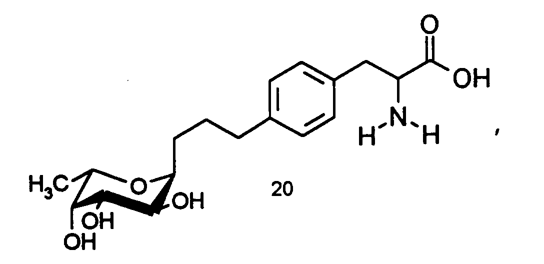
9. oder dadurch gekennzeichnet, daß

   - R⁸: eine Gruppe der Formel III 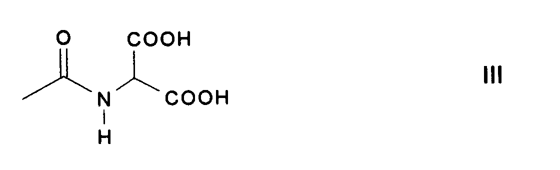 bedeutet, beispielsweise 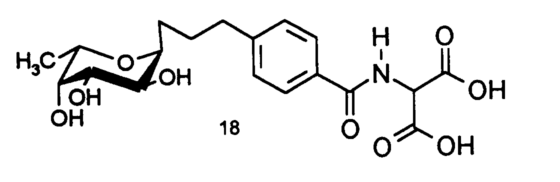
10. oder dadurch gekennzeichnet, daß

   - R⁸: eine Gruppe der Formel IV 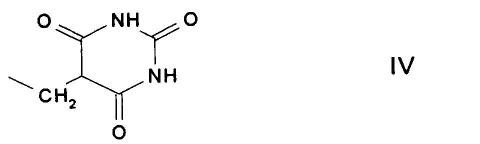 bedeutet, beispielsweise 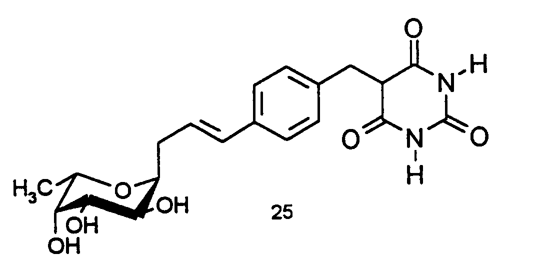
11. oder dadurch gekennzeichnet, daß

   - R⁸: eine Gruppe der Formel V 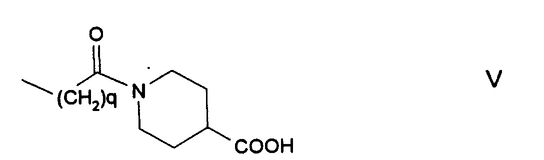 bedeutet, wobei q eine ganze Zahl von eins bis zehn darstellt, beispielsweise 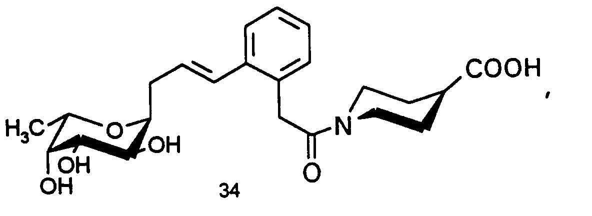
12. oder dadurch gekennzeichnet, daß

   - R⁸: eine Gruppe der Formel VI 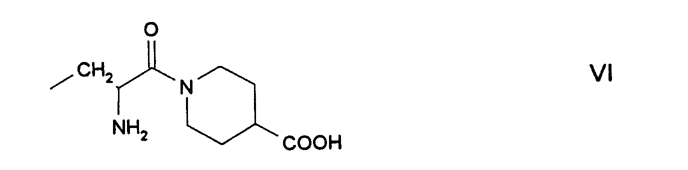 bedeutet, beispielsweise 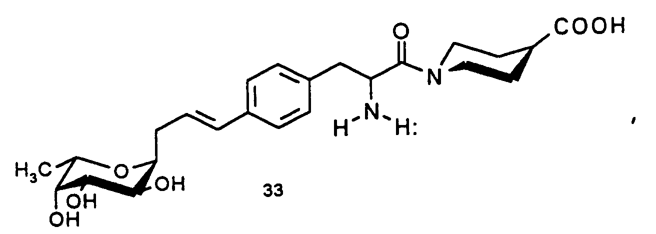
13. oder dadurch gekennzeichnet, daß

   - R⁸: eine Gruppe der Formel VII 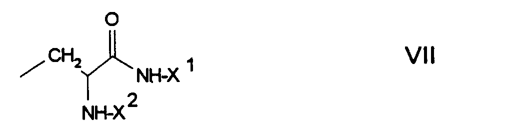 bedeutet, wobei X¹ und X² unabhängig voneinander H oder ein Oligopeptid darstellen, beispielsweise

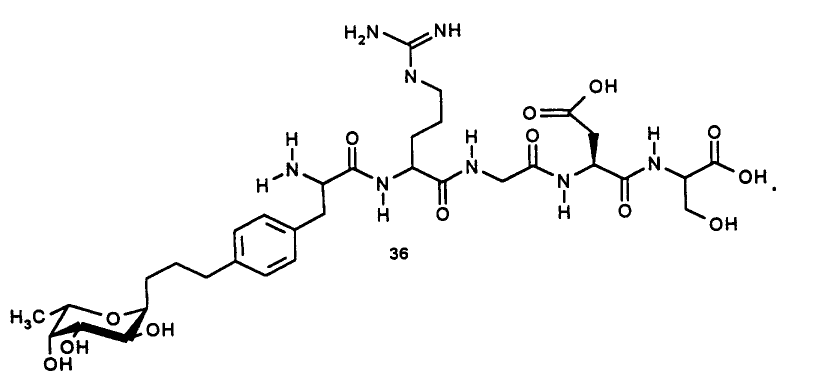

   Die Verbindung der Formel I zeichnet sich ferner vorzugsweise dadurch aus,
14. daß eine der Variablen A,B, G, E oder D CR⁹ und alle übrigen dieser Variablen C-H bedeuten,
15. besonders bevorzugt dadurch gekennzeichnet, daß R⁹ eine 4-Hydroxypiperidin-4-yl-Gruppe bedeutet, beispielsweise 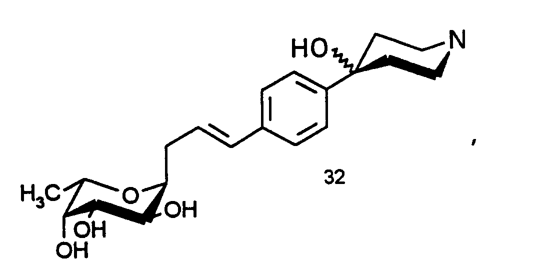
16. oder dadurch gekennzeichnet, daß die Variablen E und G gemeinsam einen substituierten Imidazolring bilden und die übrigen Variablen A, B und D C-H bedeuten, beispielsweise 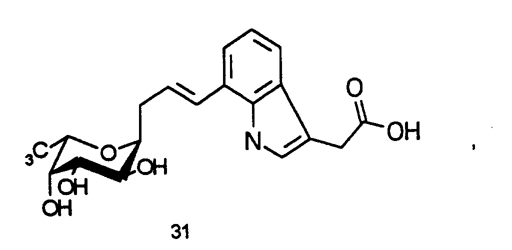
17. oder dadurch gekennzeichnet, daß die Variablen A und B C-H, die Variablen G und E C-OH und die Variable D C-COOH bedeuten, beispielsweise

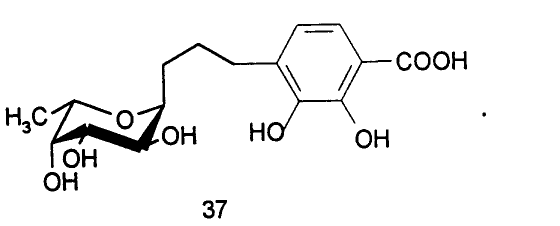

Die eingangs gestellte Aufgabe wird ferner durch ein Verfahren zur Herstellung einer Verbindung der Formel I, welches sich dadurch auszeichnet, daß man eine Verbindung der Formel VIII 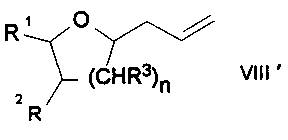 worin die Substituenten R¹, R² und R³, welche gegebenenfalls in geschützter Form vorliegen können, und die Variable n die genannten Bedeutungen haben, unter der Wirkung eines Übergangsmetallkatalysators, vorzugsweise eines Palladiumkatalysators, umsetzt mit einer Verbindung der Formel IX 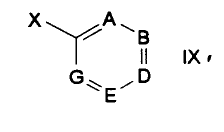 worin die Variablen A, B, D, E und G die genannten Bedeutungen haben und X ein Halogenatom darstellt und wobei die Bindung zwischen G und E auch eine Einfachbindung sein kann, wonach man das Reaktionsprodukt gegebenenfalls derivatisiert und gegebenenfalls nach Abspaltung sämtlicher Schutzgruppen die Verbindung der Formel I isoliert.

Wahlweise können bei dem Verfahren gemäß der vorliegenden Erfindung die Substituenten R¹, R² und R³ der Verbindung der Formel VIII in ungeschützter Form vorliegen.

Die Verbindungen gemäß der vorliegenden Erfindung sowie deren physiologisch verträglichen Salze eignen sich aufgrund ihrer wertvollen pharmakologischen Eigenschaften sehr gut zur Anwendung als Heilmittel bei Säugern, insbesondere dem Menschen.

Die vorliegende Erfindung betrifft daher ferner ein Arzneimittel enthaltend die Verbindung der Formel I sowie deren Verwendung für die Herstellung eines Arzneimittels zur Therapie oder Prophylaxe von Krankheiten, welche mit einer übermäßigen, durch Selektinrezeptoren vermittelten Zelladhäsion in dem von der Krankheit betroffenen Gewebe einhergehen, beispielsweise Rheuma, Reperfusionsverletzungen, Ischämie oder Herzinfarkt.

Die Arzneimittel eignen sich im besonderen zur Behandlung von akuten und chronischen Entzündungen, die sich pathophysiologisch durch eine Störung der Zellzirkulation, beispielsweise von Lymphozyten, Monozyten und neutrophilen Granulozyten, kennzeichnen lassen. Hierzu zählen Autoimmunerkrankungen wie akute Polyarthritis, rheumatoide Arthritis und Insulin-abhängige Diabetes (Diabetes mellitus DDM), akute und chronische Transplantatabstoßungen, Schocklunge (ARDS, adult respiratory distress syndrome), entzündliche und allergische Hauterkrankungen wie beispielsweise Psoriasis und Kontakt-Ekzeme, Herz-Kreislauf-Erkrankungen wie Myokardinfarkt, Reperfusionsverletzungen nach Thrombolyse , Angioplastie oder By-Pass-Operationen, septischer Schock und systemischer Schock. Eine weitere potentielle Indikation ist die Behandlung metastasierender Tumoren, denn Tumorzellen tragen Oberflächenantigene, die sowohl Sialyl-Lewis-X als auch Sialyl-Lewis-A-Strukturen als Erkennungsepitope besitzen. Darüberhinaus können diese Arzneimittel, die stabil im sauren Milieu des Magens sind, zur antiadhäsiven Therapie von Helicobacter Pylori und verwandten Mikroorganismen ggf. auch in Kombination mit Antibiotika eingesetzt werden. Ferner ist mit Hilfe dieser Arzneimittel eine Therapie der cerebralen Form der Malaria denkbar.

Die erfindungsgemäßen Arzneimittel werden im allgemeinen intravenös, oral oder parenteral oder als Implantate verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierte Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z. B. Magnesiumcarbonat` Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt.

Vorzugsweise werden die pharmazeutischen Präparate in Dosierungseinheiten hergestellt und verabreicht. Feste Dosierungseinheiten sind Tabletten, Kapseln und Suppositorien.

Für die Behandlung eines Patienten sind je nach Wirksamkeit der Verbindung, Art der Applikation, Art und Schwere der Erkrankung, Alter und Körpergewicht des Patienten, unterschiedliche Tagesdosen notwendig. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleiner Dosierungseinheiten als auch durch Mehrfachgabe unterteilten Dosen in bestimmten Intervallen erfolgen. Die zu verabreichende Tagesdosis kann außerdem von der Anzahl der während des Krankheitsverlaufs exprimierten Rezeptoren abhängig sein. Es ist vorstellbar, daß im Anfangsstadium der Krankheit nur wenige Rezeptoren auf der Zelloberfläche exprimiert werden und demzufolge die zu verabreichende Tagesdosis geringer ist als bei stark erkrankten Patienten.

Die erfindungsgemäßen Arzneimittel werden dadurch hergestellt, daß man eine Verbindunmg gemäß der vorliegenden Erfindung mit üblichen Träger- sowie gegebenenfalls Zusatz- und/oder Hilfsstoffen in die bzw. eine geeignete Darreichungsform bringt.

Im folgenden wird die Erfindung detailliert beschrieben, insbesondere in ihren bevorzugten Ausführungsformen.

### Synthese von Verbindungen der Formel I

Die Synthese des reinen α-C-Glycosids **8** konnte in nur 3 Stufen mit einer Gesamtausbeute von 72 % durchgeführt werden (Schema).

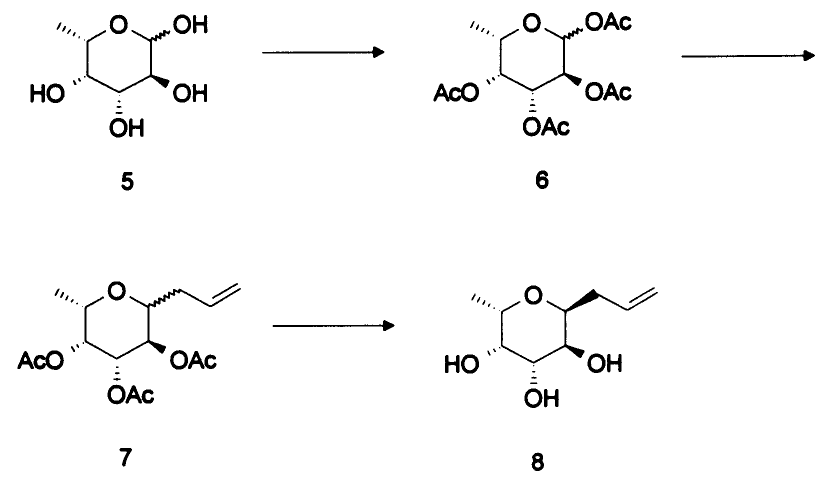

Nach Acetylierung von L-Fucose **5** mit Acetanhydrid/Pyridin (95-98 %) wurde unter BF₃-Katalyse mit einem Allylsilan umgesetzt (92 %, α/β Verhältnis 10/1). Das erhaltene C-Glycosid **7** wurde entacetyliert (quantitativ) und durch Umkristallisation gereinigt. Durch geeignete Wahl der Umkristallisationsbedingungen konnte das α-Derivat **8** im Gegensatz zu bekannten Verfahren rein erhalten werden (d.h. ohne Beimengungen des entsprechenden β-Derivats). Den einzigen Reinigungsschritt in dieser Sequenz stellt die Umkristallisation dar.

Das C-Glycosid **8** kann ohne die weitere Einführung von Schutzgruppen zur Funktionalisierung und Fixierung der Seitenkette eingesetzt werden. Wahlweise kann die perbenzylierte Verbindung **8a** eingesetzt werden.

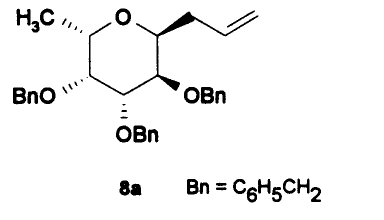

Als besonders geeignet erwies sich hierbei die übergangsmetallkatalysierte C-C-Verknüpfung eines geschützten oder auch ungeschützten C-Allylglycosids (z.B. **8** bzw. **8a**) mit einem substituierten bzw. nicht substituierten Aryl- oder Heteroarylhalogenid/triflat (die sogenannte Heck-Reaktion, s. z.B. Larock et. al., J. Org. Chem. 1991, 56, 2615). Die Synthese führt im allgemeinen in wenigen Stufen in guten bis sehr guten Ausbeuten zur pharmakologisch relevanten Endverbindung. Die Produkte der Heckreaktion lassen sich häufig ohne weitere chromatographische Reinigung durch Extraktion oder Umfällen isolieren.

Die Doppelbindung kann einfach zur weiteren Derivatisierung genutzt werden. Hydrierung, Halogenierungen, Diels-Alder-Reaktionen, Epoxydierung (Jacobsen et. al., J. Am. Chem. Soc. 1990, 112, 2801) oder Hydroxylierungen (Sharpless et. al., J. Am. Chem. Soc. 1994, 116, 1278) sowie andere Funktionalisierungen bieten einen effizienten und einfachen Zugang zu modifizierten Derivaten. So liefert zum Beispiel die Heck-Reaktion von Boc-4-Jod Phenylalanin (BOC = Benzyloxycarbonyl) mit dem Allylfucosid die entsprechende Neo-Glycoaminosäure, die sowohl in der Festphasenpeptidsynthese als auch in der flüssigen Phase zur Synthese von modifizierten Peptiden erfolgreich eingesetzt werden konnte.

In analoger Weise lassen sich die Reaktionen auf andere C-Glycosidbausteine wie beispielsweise Verbindungen **9** und **9a**, welche aus D-Mannose hergestellt werden kann, oder Verbindungen **10** bzw. **10a**, welche aus Ribose herstellbar sind, übertragen

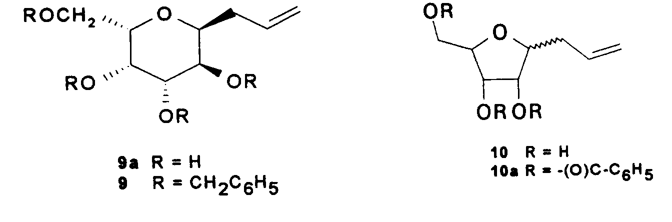

Ferner lassen sich nach dem Verfahren gemäß der vorliegenden Erfindung entsprechend Derivate der L-Galactose, L-Rhamnose und der Glucose herstellen.

Primärassays zur Untersuchung der Wirkung der Verbindungen gemäß der vorliegenden Erfindung auf die Zellanheftung an rekombinante, lösliche Selektin-Fusionsproteine.

Um die Wirksamkeit der erfindungsgemäßen Verbindungen auf die Interaktion zwischen den E- und P-Selektinen (alte Nomeklatur ELAM-1 bzw. GMP-140) mit ihren Liganden zu testen, wird ein Assay verwendet, der jeweils nur für eine dieser Interaktionen spezifisch ist. Die Liganden werden in ihrer natürlichen Form als Oberflächenstrukturen auf promyelozytischen HL60 Zellen angeboten. Da HL60 Zellen Liganden und Adhäsionsmoleküle unterschiedlichster Spezifität aufweisen, kann die gewünschte Spezifität des Assays nur über den Bindungspartner erbracht werden. Als Bindungspartner wurden gentechnisch hergestellte lösliche Fusionsproteine aus der jeweils extrazytoplasmatischen Domäne von E- bzw. P-Selektin und der konstanten Region eines humanen Immunglobulins der Subklasse IgG1 verwendet.

### Herstellung von L-Selektin-IgG1

Zur Herstellung von löslichem L-Selektion-IgG1 Fusionsprotein wurde das von Walz et al., 1990 publizierte genetische Konstrukt "ELAM-Rg" verwendet.

Zur Expression wurde die Plasmid DNA in COS-7 Zellen (ATCC) mittels DEAE-Dextran transfiziert (Molekularbiologische Methoden: siehe Ausubel, F. M., Brent, R., Kingston, R. E., Moore, D. D., Seidman, J. G., Struhl, K. und Smith, J. A. 1990. Current Protocols in Molecular Biology, John Wiley, New York). Sieben Tage nach der Transfection wird der Kulturüberstand gewonnen, durch Zentrifugation von Zellen und Zellfragmenten befreit und auf 25 mM Hepes pH 7,0, 0,3 mM PMSF, 0,02 % Natriumazid gebracht und bei +4°C aufgehoben. (Walz, G., Aruffo, A., Kolanus, W., Bevilacqua, M. und Seed, B. 1990. Recognition by ELAM-1 of the sialyl-Lex determinant on myeloid and tumor cells. Science 250, 1132-1135.)

### Herstellung von P-Selektin-IgG1

Zur Herstellung des löslichen P-Selektin-IgG1 Fusionsproteins wird das von Aruffo et al., 1991 publizierte genetische Konstrukt "CD62Rg" verwendet. Die weitere Vorgehensweise entspricht der unter A1 dargestellten Herstellung von L-Selektin-IgG1.

Aruffo, A., Kolanus, W.; Walz, G., Fredman, P. und Seed, B. 1991. CD62/-P-Selectin recognition of myeloid and tumor cell sulfatides. Cell 67, 35-44.

### Herstellung von CD4-IgG1

Zur Herstellung des löslichen CD4-IgG1 Fusionsproteins wird das von Zettlemeissl et al., 1990 publizierte genetische Konstrukt "CD4:IgG1 hinge" verwendet. Die weitere Vorgehensweise entspricht der unter A1 dargestellten Herstellung von L-Selektin-IgG1. (Zettelmeissl, G., Gregersen, J.-P., Duport, J. M., Mehdi, S., Reiner, G. und Seed, B. 1990. Expression and characterization of human CD4: Immunoglobulin Fusion Proteins. DNA and Cell Biology 9, 347-353.)

### Durchführung des HL60 Zelladhäsionsassays auf rekombinanten, löslichen Adhäsionsmolekülen

1. 96er Mikrotitertestplatten (Nunc Maxisorb) werden mit 100 µl eines in 50 mM Tris pH 9,5 verdünnten (1 + 100) Ziege anti human IgG Antikörpers (Sigma) 2 Std. bei Raumtemperatur inkubiert. Nach Entfernen der Antikörperlösung wird einmal mit PBS gewaschen.
2. 150 µl des Blockierungspuffers werden für 1 Std. bei Raumtemperatur in den Näpfchen belassen. Die Zusammensetzung des Blockierungspuffers ist: 0,1 % Gelatine, 1 % BSA, 5 % Kalbserum, 0,2 mM PMSF, 0,02 % Natriumazid. Nach Entfernen des Blockierungspuffers wird einmal mit PBS gewaschen.
3. In die Näpfchen werden je 100 µl Zellkulturüberstand von entsprechend transfektierten und exprimierenden COS-Zellen pipettiert. Die Inkubation erfolgt 2 Std. bei Raumtemperatur. Nach Entfernen des Zellkulturüberstandes wird einmal mit PBS gewaschen.
4. In die Näpfchen werden 20 µl Bindungspuffer gegeben. Der Bindungspuffer hat die Zusammensetzung: 50 mM Hepes, pH 7,5; 100 mM NaCl; 1 mg/ml BSA;
   2 mM MgCl2; 1mM CaCl2; 3 mM MnCl2; 0,02 % Natriumazid; 0,2 mM PMSF. Dazu werden 5 µl der Testsubstanz pipettiert, durch Schwenken der Platte vermischt und 10 Min. bei Raumtempeartur inkubiert.
5. 50 ml einer HL60 Zellkultur mit 200.000 Zellen/ml werden 4 Min. bei 350 g zentrifugiert. Das Pellet wird in 10 ml RPMI 1640 resuspendiert und die Zellen erneut zentrifugiert. Zur Markierung der Zellen werden 50 µg BCECF-AM (Molecular Probes) in 5 µl wasserfreiem DMSO aufgelöst; anschließend werden 1,5 ml RPMI 1640 auf die BCECF-AM/DMSO-Lösung gegeben. Mit dieser Lösung werden die Zellen resuspendiert und 30 Min. bei 37°C inkubiert. Nach zweiminütiger Zentrifugation bei 350 g wird das markierte Zellpellet in 11 ml Bindungspuffer resuspendiert und die resuspendierten Zellen in 100 µl Aliquots in die Mikrotiterplatten-Näpfchen verteilt. Die Platte wird 10 Min. bei Raumtemperatur stehen gelassen, um die Zellen auf den Boden der Testplatte sedimentieren zu lassen. Dabei haben die Zellen Gelegenheit an das beschichtete Plastik zu adhärieren.
6. Zum Abstoppen des Tests wird die Mikrotiterplatte im 45° Winkel gänzlich in den Stoppuffer getaucht (25 mM Tris, pH 7,5; 125 mM NaCl; 0,1 % BSA; 2 mM MgCl2; 1 mM CaCl2; 3 mM MnCl2; 0,02 % Natriumazid). Durch Invertierung wird der Stoppuffer aus den Näpfchen entfernt und die Prozedur noch zweimal wiederholt.
7. Die Messung der in den Näpfchen festhaftenden, BCECF-AM-markierten Zellen erfolgt in einem Cytofluorimeter (Millipore), bei einer Empfindlichkeitseinstellung von 4, einer Anregungswellenlänge von 485/22 nm und einer Emissionswellenlänge von 530/25 nm.

### Ergebnisse:

### IC₅₀-Werte für E-Selektin [mM] und für P-Selektin [mM]:

| Verbindung | E-Selektin | P-Selektin |
|---|---|---|
| 20 | 2 | 2 |
| 23 | 2 | 2 |
| 24 | 5 | 5 |
| 25 | 5 | 5 |
| 26 | 5 | 5 |
| 28 | 5 | 5 |
| 28a | 5 | 5 |
| 29 | 5 | 5 |
| 36 | 5 | 3-5 |

Leukozyten-Adhäsion - Prüfung der Wirksamkeit der erfindungsgemäßen Verbindungen in vivo

Bei entzündlichen Prozessen und anderen, die Zytokine aktivierenden Zuständen spielt die Gewebszerstörung durch einwandernde oder die Mikrozirkulation blockierende Leukozyten eine entscheidende Rolle. Die erste und für den weiteren Krankheitsprozeß entscheidende Phase ist die Aktivierung von Leukozyten innerhalb der Blutbahn, insbesondere im prä- und postkapillären Bereich. Dabei kommt es, nachdem die Leukozyten den Axialstrom des Blutes verlassen haben, zu einem ersten Anheften der Leukozyten an der Gefäßinnenwand, d.h. am Gefäßendothel. Alle darauf folgenden Leukozyteneffekte, d.h. die aktive Durchwanderung durch die Gefäßwand und die anschließende orientierte Wanderung im Gewebe, sind Folgereaktionen (Harlan, J.M., Leukocyte-endothelial interaction, Blood 65, 513-525, 1985).

Diese rezeptorvermittelte Interaktion von Leukozyten und Endothelzellen wird als ein initiales Zeichen des Entzündungsprozesses angesehen. Neben den schon physiologisch exprimierten Adhäsionsmolekülen kommt es unter der Einwirkung von Entzündungsmediatoren (Leukotriene, PAF) und Zytokinen (TNF-alpha, Interleukinen) zur zeitlich gestuften, massiven Expression von Adhäsionsmolekülen auf den Zellen. Sie werden derzeit in drei Gruppen eingeteilt: 1. Immunglobulin-Gensuperfamilie, 2. Integrine und 3. Selektine. Während die Adhäsion zwischen Molekülen der Ig-Gensuperfamilie und den Protein-Protein-Bindungen abläuft, stehen bei der Kooperation zwischen Selektinen Lektin-Kohlehydrat-Bindungen im Vordergrund (Springer, T.A., Adhesion receptors of the immune system. Nature 346, 425-434, 1990; Huges, G., Cell adhesion molecules - the key to an universal panacea, Scrips Magazine 6, 30-33, 1993; Springer, T.A., Traffic signals for lymphocyte recirculation and leukocyte emigration; The multistep paradigm. Cell 76, 301-314, 1994).

### Methode:

Die induzierte Adhäsion von Leukozyten wird mit einer intravitalmikroskopischen Untersuchungstechnik im Mesenterium der Ratte quantifiziert (Atherton A. and Born G.V.R., Quantitative investigations of the adhesiveness of circulating polymorphnuclear leukocytes to blood vessel walls. J. Physiol. 222, 447-474, 1972; Seiffge, D. Methoden zur Untersuchung der Rezeptor-vermittelten Interaktion zwischen Leukozyten und Endothelzellen im Entzündungsgeschehen, in: Ersatz- und Ergänzungsmethoden zu Tietversuchen in der biomedizinischen Forschung, Schöffl, H. et al., (Hrsg.) Springer, 1995 (im Druck)). Unter Inhalations-Äthernarkose wird eine Dauernarkose durch intramuskuläre Injektion von Urethan (1,25 mg/kg KG) eingeleitet. Nach Freipräparation von Gefäßen (V. femoralis zur Injektion von Substanzen und A. carotis zur Blutdruckmessung) werden Katheter in diese eingebunden. Danach wird das entsprechende transparente Gewebe (Mesenterium) nach den in der Literatur bekannten Standardmethoden freigelegt und auf dem Mikroskoptisch ausgelagert und mit 37°C warmen Paraffinöl überschichtet (Menger, M.D. and Lehr, H., A. Scope and perspectives of intravital microscopy-bridge over from in vitro to in vivo, Immunology Today 14, 519-522, 1993). Die Applikation der Testsubstanz erfolgt i.v. am Tier (10mg/kg). Die experimentelle Erhöhung der Blutzell-Adhäsion wird durch systemische Verabreichung von Lipopolysaccharid (LPS, 15 mg/kg) 15 Minuten nach Applikation aus Testsubstanz durch Zytokin-Aktivierung ausgelöst (Foster S.J., Mc Cormick L.M., Ntolosi B.A. and Campbell D., Production of TNF-alpha by LPS-stimulated murine, rat and human blood and its pharmacological modulation, Agents and Actions 38, C77-C79, 1993, 18.01.1995). Die dadurch verursachte erhöhte Adhäsion von Leukozyten am Endothel wird direkt vitalmikroskopisch oder mit Hilfe von Fluoreszenzfarbstoffen quantifiziert. Alle Meßvorgänge werden per Videokamera aufgenommen und auf einem Videorekorder gespeichert. Über einen Zeitraum von 60 Minuten wird alle 10 Minuten die Anzahl der rollenden Leukozyten (d.h. alle sichtbar rollenden Leukozyten, die langsamer als die strömenden Erythrozyten sind) und die Anzahl an haftenden Leukozyten am Endothel (Verweildauer länger als 5 Sekunden) erfaßt. Nach Beendigung des Versuches werden die narkotisierten Tiere schmerzfrei durch systemische Injektion von T61 exzitationsfrei eingeschläfert. Zur Auswertung werden die Ergebnisse jeweils von 8 behandelten mit 8 unbehandelten Tieren (Kontrollgruppe) verglichen (in Prozenten).

### Beispiele

### I. Darstellung der C-Glycosidbausteine bzw. der Verbindungen der Formel VIII

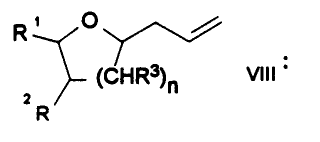

### Beispiel 1

### Darstellung des C-Glycosidbausteins 8 (L-Fucosederivat)

L-Fucose (5) (250 g, 1.52 mol) wird mit Pyridin (616 ml, 7.62 mol) und Acetanhydrid (633 ml, 6.7 mol) versetzt und 24 h bei Raumtemperatur gerührt. Das Lösungsmittel wird am Rotationsverdampfer entfernt und der Rückstand am Hochvakuum getrocknet. Man erhält 6 (496 g, 98 %) als gelbes Öl. DC [Hexan/Essigsäureethylester : 1/1]: R_{f} = 0.60. - ¹H-NMR (300 MHz, CDCl₃): δ = 1.18 (d, 3 H, J_{6,5} = 6.9 Hz, 6-H^{Fuc}), 2.00-2.15 (4 s, 12 H, CH₃),

Das Fucosederivat 6 (50 g, 0.15 mol) wird unter Argon in Acetonitril (300 ml) gelöst und auf -10 °C abgekühlt. Nach Zugabe von Allylsilan (47.9 ml, 0.30 mol) und Bortrifluorid - Diethylether Komplex (20.4 ml, 0.166 mol) wird das Gemisch 1 h bei -10 °C gerührt. Nach Erwärmen auf Raumtemperatur wird weitere 3 h gerührt. Die Lösung wird auf gesättigte Natriumhydrogencarbonatlösung gegeben und mit Essigester extrahiert. Das Lösungsmittel wird am Rotationsverdampfer entfernt. Der verbleibende Rückstand wird in Dichlormethan aufgenommen und über eine kurze Kieselgelsäule filtriert. Nach Entfernen des Lösungsmittels erhält man 7 (α/β 10/1) als gelbes Öl (43.5 g, 92 %).
DC [Hexan/Essigsäureethylester : 1/1]: R_{f} = 0.65 - ¹H-NMR (300 MHz, CDCl₃): δ = 1.19 (d, 3 H, J_{6,5} = 6.9 Hz, 6-H^{Fuc}), 2.00-2.15 (3 s, 9 H, CH₃), 2.20-2.58 (m, 2H), 3.98 (m, 1H), 4.28 (m, 1H), 5.05-5.85 (m, 6H).

Das Allylderivat 7 (43.5 g, 0.139 mol) wird in Methanol (200 ml) gelöst und mit Natriummethanolatlösung (3 ml, 30 %ig) versetzt. Die Lösung wird bei Raumtemperatur 2 h gerührt, mit saurem Ionenaustauscher (Dowex® 50 W X 8) neutralisiert. Der Ionenaustauscher wird abfiltriert und das Lösungsmittel am Rotationsverdampfer abgezogen. Man erhält das entacetylierte Fucosederivat als leicht gelben Feststoff (26.0 g, quantitativ).
Der Rückstand wird in Essigester in der Wärme gelöst und heiß filtriert. Nach Entfernen des Lösungsmittels am Rotationsverdampfer wird der Rückstand in feuchtem Essigester in der Wärme erneut gelöst. Beim Abkühlen der Lösung kristallisiert 8 als weißer Feststoff aus (20.8 g, 80 %).
DC [Dichlormethan/Methanol: 10/1]: R_{f} = 0.25 - ¹H-NMR (300 MHz, D₂O): δ = 1.05 (d, 3 H, J_{6,5} = 6.9 Hz, 6-H^{Fuc}), 2.19-2.43 (m, 2H), 3.60-4.00 (m, 5 H), 5.05 (m, 2 H), 5.62-5.80 (m, 1H).
Unter Standardbedingungen (s. Masamune et. al., Tetrahedron Lett. 1987, 28, 4303) kann 8 in das entsprechende Tribenzylderivat 8a überführt werden.

### Beispiel 2

### Darstellung des C-Glycosidbausteins 9 (Mannosederivat)

Analog zu 8 wird das Mannosederivat 9 als α/β Gemisch über 3 Stufen in 62 % Ausbeute dargestellt.

DC [Dichlormethan/Methanol: 10/1]: R_{f} = 0.30 - ¹H-NMR (300 MHz, D₂O): δ = 2.19-2.43 (m, 2H), 3.60-4.00 (m, 7 H), 5.05 (m, 2 H), 5.62-5.80 (m, 1H).

### Beispiel 3

### Darstellung des C-Glycosidbausteins 10a (Ribosederivat)

1-O-acetyl-2,3,5-tri-O-benzoyl-β-D-ribofuranose (1.0 g, 2.0 mmol) wird unter Argon in Acetonitril (10 ml) gelöst und auf -10 °C abgekühlt. Nach Zugabe von Allylsilan (0.48 ml, 3.0 mmol) und Bortrifluorid - Diethylether Komplex (0.25 ml, 0.2 mmol) wird das Gemisch 1 h bei -10 °C gerührt. Nach Erwärmen auf Raumtemperatur wird weitere 2 h gerührt. Die Lösung wird auf gesättigte Natriumhydrogencarbonatlösung gegeben und mit Essigester extrahiert. Das Lösungsmittel wird am Rotationsverdampfer entfernt. Der verbleibende Rückstand wird in Dichlormethan aufgenommen und über eine kurze Kieselgelsäule filtriert. Nach Entfernen des Lösungsmittels erhält man die Allylverbindung 10a (α/β 4/1) als gelbes Öl (0.798 g, 76 %).

DC [Hexan/Essigsäureethylester : 1/1]: R_{f} = 0.60 - ¹H-NMR (300 MHz, CDCl₃): δ = 2.51 (m, 2 H), 4.20-6.05 (m, 8 H), 7.10-8.35 (m, 15 H).

### II. Heck-Reaktionen mit geschützten Allyl-C-Glycosiden, Umsetzungen der geschützten Verbindungen der Formel VIII mit Verbindungen der Formel IX

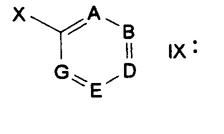

### Beispiel 4

Eine Mischung von 2,3,4-Tri-O-benzyl-C-allyl-fucopyranosid 8a (200 mg, 0.436 mmol), Iodbenzol (89 mg, 0.436 mmol) und Natriumcarbonat (69 mg, 0.651 mmol) in Dimethylformamid wird unter Argonatmosphäre mit Pd(OAc)₂ (3 mg, 3 mol/%) versetzt und bei einer Temperatur von 70°C wird für 16 h gerührt. Man filtriert, entfernt die Lösungsmittel am Hochvakuum und erhält nach Flashchromatographie an Kieselgel mit i-Hexan/ Essigsäureethylester (10/1) 11 als farbloses Öl (190 mg, 0.355 mmol, 81.5%).
DC [Hexan/Essigsäureethylester : 3/1]: R_{f} = 0.56. - ¹H-NMR (300 MHz, CDCl₃): δ = 1.32 (d, 3 H, J_{6,5} = 6.9 Hz, 6-H^{Fuc}), 2.49 (m, 2 H, α-CH₂), 3.83-3.86, 4.01, 4.12 (5 H, 1-H^{Fuc}, 2-H^{Fuc}, 3-H^{Fuc}, 4-H^{Fuc}, 5-H^{Fuc}), 4.42-4.80 (6 H, CH₂-Ph), 6.11 (dt, 1 H, J_{β,α} = 7.1, J_{β,γ} = 15.8 Hz , β-H), 6.39 (d, 1 H, J_{γ,β} = 15.9 Hz , γ-H), 7.23-7.40 (20 H, H-Ar);
¹³C-NMR (75.4 MHz, CDCl₃): d = 15.11 (6-C^{Fuc}), 31.96 (a-CH₂), 126.05-128.27, 128.34, 128.37, 128.40 (C-Ar), 131.72 (C-Allyl), 137.62, 138.29, 138.59, 138.78 (C_{quart}.-Ar).

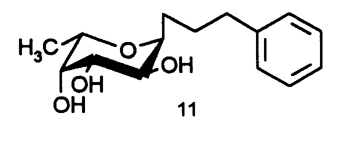

### Beispiel 5

Eine Mischung von 2,3,4-Tri-O-benzyl-C-allyl-fucopyranosid 8a (185 mg, 0.403 mmol), 4'-Brombenzo-18-Krone-6 (189 mg, 0.484 mmol) und Natriumcarbonat (106 mg, 1 mMol) in Dimethylformamid wird unter Argonatmosphäre mit Pd(OAc)₂ (4.5 mg, 5 mol/%) versetzt und bei einer Temperatur von 70°C für 3 h gerührt. Nach Zugabe von (21 mg, 20 mol/%) Triphenylphosphin wird für weitere 6 h bei 100°C gerührt, anschließend filtriert und die Lösungsmittel am Hochvakuum entfernt. Nach Flashchromatographie an Kieselgel mit Dichlormethan/Methanol (15/1) erhält man 12 als amorphen Feststoff (206 mg, 0.268 mmol, 66.5 %).
DC [Dichlormethan/Methanol : 10/1]: R_{f} = 0.25. - ¹H-NMR (300 MHz, CDCl₃): δ = 1.32 (d, 3 H, J_{6,5} = 6.7 Hz, 6-H^{Fuc}), 2.46 (m, 2 H, α-CH₂), 3.6-3.9 (m, H-Kron-6), 3.6-4.2 (5 H, 1-H^{Fuc}, 2-H^{Fuc}, 3-H^{Fuc}, 4-H^{Fuc} , 5-H^{Fuc}), 4.44-4.80 (6 H, CH₂-Ph), 5.98 (dt, 1 H, J_{β,α} = 7., J_{β,γ} = 16 Hz , β-H), 6.30 (d, 1 H, J_{γ,β} = 16 Hz , γ-H), 6.9-7.40 (18 H, H-Ar).

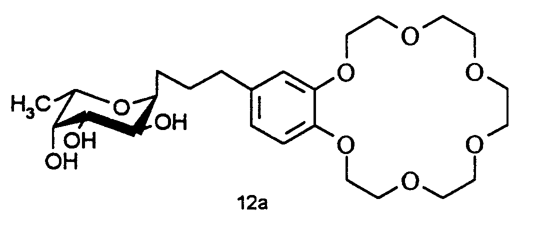

### Beispiel 6

Eine Lösung von 12 (153 mg, 0.199 mmol) in einer Mischung aus Methanol/Dioxan/AcOH (10/1/1, 10 ml) versetzt man mit Pd/C (10% Pd auf Aktivkohle, 100 mg) und hydriert 16 h unter Wasserstoffatmosphäre. Der Ansatz wird mit 20 ml Methanol versetzt, durch einen sterilen Spritzenfilter (0.2 mm, Schleicher & Schuell) filtriert und die Lösung i. Vak. eingeengt. Ausschlußchromatographie an Sephadex LH20 mit Methanol als Eluent führt zu 12a (87 mg, 0.173 mmol, 87%) als amorphem Feststoff.

DC [Butanol/Essigsäure/Wasser : 3/1/1]: R_{f} = 0.17. - ¹H-NMR (300 MHz, CD₃OD): δ = 1.23 (d, 3 H, J_{6,5} = 6.6 Hz, 6-H^{Fuc}), 1.64 (m, 4 H, β,γ-CH₂), 2.63 (m, 2 H, α-CH₂), 3.67, 3.88, 4.14 (m, H-Kron-6, 1-H^{Fuc}, 2-H^{Fuc}, 3-H^{Fuc}, 4-H^{Fuc}, 5-H^{Fuc}), 6.94-6.72 (18 H, H-Ar).

### Beispiel 7

Eine Lösung von 2,3,4-Tri-O-benzyl-C-allyl-fucopyranosid 8a (100 mg, 0.218 mmol), 4-Iodanisol (51 mg, 0.218 mmol) und Triethylamin (44.1 mg, 0.436 mmol) in Dimethylformamid wird unter Argonatmosphäre mit Pd(OAc)₂ (3 mg, 3 mol/%) versetzt. Es wird bei einer Temperatur von 100°C für 16 h gerührt. Man filtriert, entfernt die Lösungsmittel am Hochvakuum und erhält nach Flashchromatographie an Kieselgel mit i-Hexan/ Essigsäureethylester (8/1) 13 als farbloses Öl (45 mg, 0.08 mmol, 36.6 %).
DC [Hexan/Essigsäureethylester : 4/1]: R_{f} = 0.36. - ¹H-NMR (300 MHz, CDCl₃): δ = 1.31 (d, 3 H, J_{6,5} = 6.6 Hz, 6-H^{Fuc}), 2.47 (m, 2 H, α-CH₂), 3.80, 4.01, 4.10 (5 H, 1-H^{Fuc}, 2-H^{Fuc}, 3-H^{Fuc}, 4-H^{Fuc}, 5-H^{Fuc}), 3.81 (s, 3 H, OCH₃), 4.42-4.80 (6 H, CH₂-Ph), 5.96 (dt, 1 H, J_{b,a} = 7.1, J_{b,g} = 15.8 Hz, β-H], 6.34 (d, 1 H, J_{γ,β} = 15.8 Hz , γ-H), 6.88 (d, 2 H, J_{Ar} = 8.9 Hz, Ar), 7.22 (d, 2 H, J_{Ar} = 8.9 Hz, Ar), 7.24-7.40 (15 H, H-Ar).

### Beispiel 8

Eine Lösung von 13 (31 mg, 0.055 mmol) in Methanol/Ameisensäure (10 ml/5 ml) versetzt man mit Pd_{black} (200 mg). Nach 16 h wird filtriert und die Lösung i. Vak. eingeengt. Ausschlußchromatographie an Biogel P2 führt zu 14 (15 mg, 0.05 mmol, 91 %) als amorphem Feststoff.

¹H-NMR (300 MHz, CD₃OD): δ = 1.23 (d, 3 H, J_{6,5} = 6.6 Hz, 6-H^{Fuc}), 1.64 (m, 4 H, β,γ-CH₂), 2.63 (m, 2 H, α-CH₂), 6.9-7.3(4 H, H-Ar).

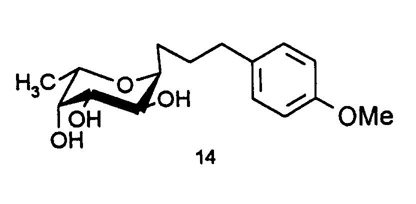

### Beispiel 9

Eine Mischung von 2,3,4-Tri-O-benzyl-C-allyl-fucopyranosid 8a (250 mg, 0.545 mmol), 4-Brombenzoesäureethylester (137 mg, 0.6 mmol) und Natriumcarbonat (116 mg, 1.09 mmol) in Dimethylformamid (10 ml) wird unter Argonatmosphäre mit Pd(OAc)₂ (3.7 mg, 3 mol/%) versetzt. Es wird bei einer Temperatur von 100°C für 24 h gerührt. Man filtriert, entfernt die Lösungsmittel am Hochvakuum und erhält nach Flashchromatographie an Kieselgel mit Dichlormethan/Methanol (8/1) 15 als amorphen Feststoff (284 mg, 0.468 mmol, 85.9%)
DC [Hexan/Essigsäureethylester : 3/1]: R_{f} = 0.34.

### Beispiel 10

Eine Lösung von 15 (242 mg, 0.399 mmol) in Methanol/Dioxan/Ameisensäure (10 ml/1 ml/1 ml) versetzt man mit Pd_{black} (200 mg). Man filtriert nach 24 h, entfernt die Lösungsmittel i. Vak. und löst den Rückstand mit Methanol/Wasser/1 M NaOH (4 ml : 4 ml : 3 ml). Nach Neutralisation mit Amberlite IR 120, Filtration und Ausschlußchromatographie an Sephadex® LH20 mit Methanol als Eluent erhält man 16 (120 mg, 0.387 mmol, 97%) als amorphen Feststoff.
DC [RP 18, Wasser/Methanol : 1/1]: R_{f} = 0.30. - ¹H-NMR (300 MHz, CDCl₃): δ = 1.14 (d, 3 H, J_{6,5} = 6.4 Hz, 6-H^{Fuc}), 1.52 (β- oder γ-CH₂), 1.68 (α-CH₂), 2.62-2.83 (β- oder γ-CH₂), 3.64-3.73 (3-H^{Fuc}, 4-H^{Fuc}, 5-H^{Fuc}), 3.92 (2-H^{Fuc}), 3.98 (1-H^{Fuc}), 7.35 (d, 1 H, J_{Ar} = 8 Hz, H-Ar), 7.84 (d, 1 H, J_{Ar} = 8 Hz, H-Ar); ¹³C-NMR (75.4 MHz, CDCl₃): δ = 18.4 (6-C^{Fuc}), 25.5, 29.5, 37.4 (α-C, β-C, γ-C), 69.5 (3-C^{Fuc} oder 4-C^{Fuc} oder 5-C^{Fuc}), 70.9 (2-C^{Fuc}), 72.7 (3-C^{Fuc} oder 4-C^{Fuc} oder 5-C^{Fuc}), 74.6 (3-C^{Fuc} oder 4-C^{Fuc} oder 5-C^{Fuc}), 78.4 (1-C^{Fuc}), 131.2, 132 (C-Ar), 136.5, 149.2 (C_{quart}.-Ar), 178.2 (C=O).

### Beispiel 11

Aminomalonsäurediethylester (17.2 mg, 0.081 mmol) und 16 (18 mg, 0.058 mmol) werden in Dimethylformamid gelöst. Man kühlt den Ansatz auf 0°C, gibt N-(3-Dimethylaminopropyl)-N'-ethylcarbodiimid hydrochlorid zu, läßt 1h bei 0°C rühren und erwärmt binnen 2 h auf Raumtemperatur. Die Lösungsmittel werden i. Vak. entfernt und der Rückstand durch Flashchromatographie an Kieselgel mit Dichlormethan/Methanol (10/1) als Eluent gereinigt. Man erhält 17 (23 mg, 0.0492 mmol, 84.8%) als amorphen Feststoff.
DC [Dichlormethan/Methanol : 10/1]: R_{f} = 0.18. - ¹H-NMR (300 MHz, CD₃OD): δ = 1.10 (d, 3 H, J_{6,5} = 6.4 Hz, 6-H^{Fuc}), 1.20 (dd, 6 H, J = 7.3 Hz, CH₃), 1.53, 1.65, 2.62 (α-CH₂, β-CH₂, γ-CH₂), 3.45-4.28 (2-H^{Fuc}, 3-H^{Fuc}, 4-H^{Fuc}, 5-H^{Fuc}), 4.17 (m, 4 H, CH₂), 7.23 (d, 1 H, J_{Ar} = 8,2 Hz, H-Ar), 7.70 (d, 1 H, J_{Ar} = 8.2 Hz, H-Ar).

### Beispiel 12

Verbindung 17 (19 mg, 0.0406 mmol) wird in Methanol/ 1 M NaOH (1 ml : 3 ml) binnen 2h verseift. Nach Neutralisation mit Amberlite IR 120, Filtration und Ausschlußchromatographie an Biogel P2 mit Wasser als Eluent erhält man 18 (15 mg, 0.037 mmol, 91 %) als amorphen Feststoff.

DC [Butanol/Essigsäure/Wasser : 3/1/1]: R_{f} = 0.31. - ¹H-NMR (300 MHz, D₂O): δ = 1.08 (d, 3 H, J_{6,5} = 6.7 Hz, 6-H^{Fuc}), 1.34-1.7, 2.59-2.77 (α-CH₂, β-CH₂, γ-CH₂), 3.63-3.66, 3.82-3.94 (1-H^{Fuc}, 2-H^{Fuc}, 3-H^{Fuc}, 4-H^{Fuc}, 5-H^{Fuc}), 7.35 (d, 1 H, J_{Ar} = 8.3 Hz, H-Ar), 7.73 (d, 1 H, J_{Ar} = 8 Hz, H-Ar).

### Beispiel 13

Eine Mischung von 2,3,4-Tri-O-benzyl-C-allyl-fucopyranosid (4.34 g, 9.46 mmol), Boc-4-Jod Phenylalanin (4.47 g, 11.36 mmol) und Natriumcarbonat (6.02 g, 56.76 mmol) in Dimethylformamid (40 ml) wird unter Argonatmosphäre mit Pd(dba)₂ (3 mol/%) versetzt. Es wird bei einer Temperatur von 75°C für 6 h gerührt. Man filtriert, entfernt die Lösungsmittel am Hochvakuum und erhält nach Ausschlußchromatographie an Sephadex LH20 mit Methanol als Eluent 19 als amorphen Feststoff (5.25 g, 7.26 mmol, 76.7%).
DC [Dichlormethan/Methanol : 10/1]: R_{f} = 0.21. - ¹H-NMR (300 MHz, DMSO): δ = 1.23 (d, 3 H, J_{6,5} = 6.5 Hz, 6-H^{Fuc}), 1.39 (s, 9 H, H^{Boc}), 2.48 (m, 2 H, α-CH₂), 2.96 (dd, 1 H, J_{β,α} = 5, J_{β,β} = 14 Hz, b_{α}-H^{Phe}), 3.08(dd, 1 H, J_{β,α} = 5, J_{β,β} = 14 Hz, b_{β}-H^{Phe}), 3.65-4.1 (6 H, 1-H^{Fuc}, 2-H^{Fuc}, 3-H^{Fuc}, 4-H^{Fuc}, 5-H^{Fuc}, α-H^{Phe}), 4.46-4.79 (6 H, CH₂-Ph), 5.83 (d, 1 H, J_{NH,a} = 6 Hz, HN^{Phe}), 6.13 (dt, 1 H, J_{β,α} = 7, J_{β,γ} = 16 Hz, β-H), 6.41 (d, 1 H, J_{γ,β} = 16 Hz, γ-H), 7.09 (d, 2 H, J_{Ar} = 8 Hz, Ar), 7.20 (d, 2 H, J_{Ar} = 8 Hz, Ar), 7.30-7.45 (15 H, H-Ar).

### Beispiel 14

Man versetzt 19 (155 mg, 0.214 mmol) mit Trifluoressigsäure (20 ml, 95%) und läßt für 1 h Rühren. Nach Entfernen der Lösungsmittel i. Vak. wird der Rückstand in Methanol/Dioxan/Essigsäure (15 ml / 3 ml / 3 ml) gelöst, mit Pd/C (10% Pd auf Aktivkohle, 110 mg) versetzt und 16 h unter Wasserstoffatmosphäre hydriert. Der Ansatz wird mit Methanol (20 ml) verdünnt, filtriert und i. Vak. eingeengt. Ausschlußchromatographie an Biogel P2 mit Wasser als Eluent führt zu 20 (60 mg, 0.17 mmol, 79.2%) als amorphem Feststoff.
DC [Butanol/Essigsäure/Wasser : 3/1/1]: R_{f} = 0.34. - ¹H-NMR (300 MHz, D₂O): δ = 1.03 (d, 3 H, J_{6,5} = 6.5 Hz, 6-H^{Fuc}), 1.50-1.55 (α-CH₂, β- oder γ-CH₂), 2.59 (β-oder γ-CH₂), 2.91 (dd, 1 H, J_{β,α} = 8.3, J_{β,β} = 14.3 Hz, b_{α}-H^{Phe}), 3.20 (dd, 1 H, J_{β,α} = 4.3, J_{β,β} = 14.4 Hz, b_{β}-H^{Phe}), 3.45-3.70 (6 H, 1-H^{Fuc}, 2-H^{Fuc}, 3-H^{Fuc}, 4-H^{Fuc}, 5-H^{Fuc}, a-H^{Phe}), 7.03 (d, 2 H, J_{Ar} = 8.1 Hz, Ar), 7.11 (d, 2 H, J_{Ar} = 8.1 Hz, Ar); ¹³C-NMR (75.4 MHz, D₂O): δ = 16.1 (6-C^{Fuc}), 24.9, 27.8, 34.8 (α-C, β-C, γ-C], 36.8, 55.6, 67.5, 68.7, 70, 70.9, 72.9 (1-C^{Fuc} ,2-C^{Fuc}, 3-C^{Fuc}, 4-C^{Fuc}, 5-C^{Fuc}), 128.2, 129.2 ((C-Ar), 134.9, 140.5 (C_{quart}.-Ar), 170.1 (C=O).

### Beispiel 15

Eine Mischung von 2,3,5-tri-O-benzoyl-C-allyl-ribosid 10a (100 mg), p-Iodbenzoesäure (1.5 Äquiv) und Natriumcarbonat (3 Äquiv.) in Dimethylformamid (5 ml) wird unter Argonatmosphäre mit Pd(OAc)₂ (3 mol/%) versetzt und bei einer Temperatur von 75°C für 48 h gerührt. Man entfernt die Lösungsmittel am Hochvakuum, löst den Rückstand in wenig Methanol, fügt 5 ml Natriummethanolat in Methanol zu, läßt für 1 h rühren, neutralisiert mit saurem Ionenaustauscher Amberlite IR 120 und erhält nach Flashchromatographie an Kieselgel mit Dichlormethan/Methanol (5/1) 21 als amorphen Feststoff (25 mg).
DC [Dichlormethan/Methanol : 2/1]: R_{f} = 0.3.

### Beispiel 16

Eine Mischung von 2,3,4-Tri-O-benzyl-C-allyl-mannosid 9 (500 mg, 1.34 mmol), p-Iodbenzoesäure (432 mg, 1.74 mmol) und Natriumcarbonat (222 mg, 2.68 mmol) in Dimethylformamid (10 ml) wird unter Argonatmosphäre mit Pd(OAc)₂ (3 mol/%) versetzt und bei einer Temperatur von 80°C für 48 h gerührt. Man entfernt die Lösungsmittel am Hochvakuum und erhält nach Flashchromatographie an Kieselgel mit Dichlormethan/Methanol (15/1) 22 als amorphen Feststoff (390 mg, 59 %).

DC [Dichlormethan/Methanol : 20/1]: R_{f} = 0.15. - ¹H-NMR (300 MHz, CD₃OD): δ=1.89, 2.02, 2.07, 2.10 (s, 12 H, OAc), 2.60, 2.74 (m, 2 H, α-CH₂), 4.0-4.4 (4 H, 1-H^{Man}, 5-H^{Man}, 6-H^{Man}), 5.16, 5.25, 5.36 (3 H, 2-H^{Fuc}, 3-H^{Fuc}, 4-H^{Fuc}), 6.38 (1 H, β-H), 6.61 (d, 1 H, J_{γ,β} = 16 Hz , γ-H), 7.48 (d, 2 H, H-Ar), 7.95 (d, 2 H, H-Ar).

Katalytische Hydrierung von 22 analog Beispiel 14 führt zur entschützten Verbindung 22a.

### III. Heck-Reaktionen mit ungeschützten Allyl-C-Glycosiden, Umsetzungen der ungeschützten Verbindungen der Formel VIII mit Verbindungen der Formel IX

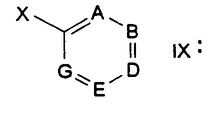

### Beispiel 17

Eine Mischung von C-allyl-fucopyranosid 8 (3 g, 15.94 mmol), 3-Jodbenzoesäure (4.74 g, 19.13 mmol) und Natriumcarbonat (5.07 g, 47.82 mmol) in Dimethylformamid (20 ml) unter Argonathmosphäre wird nach dreimaligem Entgasen mit Pd(OAc)₂ (107 mg, 3 mol/%) versetzt. Man erhöht binnen 3 h die Temperatur von 50°C auf 70°C, filtriert anschließend durch Kieselgur und entfernt die Lösungsmittel i. Vak.. Der Rückstand wird in wenig Wasser aufgenommen, durch einen sterilen Spritzenfilter (0.2 mm, Schleicher & Schuell) filtriert und zuletzt mittels Ausschlußchromatographie an Biogel® P2 mit Wasser als Eluent gereinigt. Umfällen aus Wasser mit Aceton liefert 29 als amorphen Feststoff (4.64 g, 15.04 mmol, 94.4%).
DC [Dichlormethan/Methanol : 10/1]: R_{f} = 0.49. - ¹H-NMR (300 MHz, D₂O): δ = (d, 3 H, J_{6,5} = 6.4 Hz, 6-H^{Fuc}), 2.32 (m, 2 H, α-CH₂), 3.53-3.97 (5 H, 1-H^{Fuc}, 2-H^{Fuc}, 3-H^{Fuc}, 4-H^{Fuc}, 5-H^{Fuc}), 6.08 (dt, 1 H, J_{β,α} = 7, J_{β,γ} = 15.7 Hz, β-H), 6.37 (d, 1 H, J_{γ,β} = 15.7 Hz , γ-H), 7.26 (dd, 1 H, J_{Ar} = 7.7 Hz, Ar), 7.37 (d, 1 H, J_{Ar} = 7.7 Hz, Ar), 7.59 (d, 1 H, J_{Ar} = 7.6 Hz, Ar), 7.75 (s, 1 H, Ar).

### Beispiel 18

Eine Mischung von C-allyl-fucopyranosid 8 (380 mg, 2.02 mmol), 4-Brombenzylmalonsäure Dimethylester (608 mg, 2.02 mmol) und Natriumcarbonat (642 mg, 6.06 mmol) in Dimethylformamid (10 ml) wird mit Pd(OAc)₂ (3 mol/%) versetzt. Es wird für 4 h gerührt, wobei man allmählich von 40°C auf 80°C erwärmt. Man läßt bei Raumtemperatur über Nacht rühren, filtriert und entfernt die Lösungsmittel i.Vak. Der Rückstand wird in Methanol/ 1 M NaOH (1 ml : 3 ml) binnen 1.5 h verseift. Nach Neutralisation mit Amberlite IR 120, Filtration durch einen sterilen Spritzenfilter (0.2 mm, Schleicher & Schuell) und Ausschlußchromatographie an Biogel® P2 mit Wasser als Eluent erhält man 24 als amorphen Feststoff (305 mg, 0.802 mmol, 39.7%). ¹H-NMR (300 MHz, D₂O): δ = 1.23 (d, 3 H, J_{6,5} = 6.5 Hz, 6-H^{Fuc}), 2.60 (m, 2 H, α-CH₂), 3.82-4.28 (5 H, 1-H^{Fuc}, 2-H^{Fuc}, 3-H^{Fuc}, 4-H^{Fuc}, 5-H^{Fuc}), 6.39 (ddd, 1 H, J_{β,α} = 6.5, J_{β,γ} = 15. Hz, β-H), 6.62 (d, 1 H, J_{g,b} = 15 Hz , γ-H), 7.34 (dd, 2 H, J_{Ar} = 8 Hz, Ar), 7.48 (d, 2 H, J_{Ar} = 8 Hz, Ar).

### Beispiel 19

Eine Mischung von C-allyl-fucopyranosid 8 (330 mg, 1.75 mmol), p-Brombenzylbarbitursäure (520 mg, 1.75 mmol) und Natriumcarbonat (556 mg, 5.25 mmol) in Dimethylformamid (10 ml) wird unter Argonatmosphäre mit Pd(OAc)₂ (3 mol/%) versetzt. Es wird bei einer Temperatur von 40°C- > 75°C für 4 h gerührt. Man filtriert, entfernt die Lösungsmittel i.Vak. und erhält nach Ausschlußchromatographie an Biogel® P2 mit Wasser als Eluent 25 als amorphen Feststoff (560 mg, 1.49 mmol, 85%).
DC [Dichlormethan/Methanol : 2/1]: R_{f} = 0.18. - ¹H-NMR (300 MHz, D₂O): δ = 0.99 (d, 3 H, J_{6,5} = 6.6 Hz, 6-H^{Fuc}), 2.35 (m, 2 H, α-CH₂), 3.54-4.0 (5 H, 1-H^{Fuc}, 2-H^{Fuc}, 3-H^{Fuc}, 4H^{Fuc}, 5-H^{Fuc}), 6.03 (ddd, 1 H, J_{β,α} = 7, J_{β,γ} = 16 Hz, β-H), 6.33 (d, 1 H, J_{γ,β} = 16 Hz, γ-H), 7.05 (dd, 2 H, J_{Ar} = 8 Hz, Ar), 7.18 (d, 2 H, J_{Ar} = 8 Hz, Ar); ¹³C-NMR (75.4 MHz, D₂O): δ = 15.5 (6-C^{Fuc}), 27.3, 27.8, 66.9, 67.9, 69.8, 71.8, 75.4, 89.0, 125.9, 128.1 (C-Ar), 131.8, 134.7, 141.4, 153.1, 166.4.

### Beispiel 20

Eine Mischung von C-allyl-fucopyranosid 8 (200 mg, 1.063 mmol), 5-Bromnikotinsäure (215 mg, 1.063 mmol) und Natriumcarbonat (450 mg, 4.25 mmol) in Dimethylformamid (4 ml) wird unter Argonatmosphäre mit Pd(OAc)₂ / Triphenylphosphin (3 mol/%) versetzt. Es wird bei einer Temperatur von 50°C für 4 h gerührt. Nach Zugabe von 6 ml Ethylenglycol wird die Temperatur auf 170 °C erhöht und die Suspension für 4 h gerührt. Man entfernt die Lösungsmittel i. Vak. und erhält nach Ausschlußchromatographie an Biogel® P2 mit Wasser als Eluent 26 als amorphen Feststoff (93 mg, 0.301 mmol, 28 %). DC [Dichlormethan/Methanol : 2/1]: R_{f} = 0.28. - ¹H-NMR (300 MHz, D₂O): δ = 1.0 (d, 3 H , J_{6,5} = 6.4 Hz, 6-H^{Fuc}), 1.38-1.61 (m, 4 H, β- oder γ-CH₂, α-CH₂), 2.50-2.66 (β- oder γ-CH₂), 3.44-3.89 (1-H^{Fuc}, 2-H^{Fuc}, 3-H^{Fuc}, 4-H^{Fuc}, 5-H^{Fuc}), 7.92-8.89 (d, 1 H, J_{Ar} = 8 Hz, H-Ar), 7.84 (H-Ar); ¹³C-NMR (75.4 MHz, CDCl₃): δ = 15.6 (6-C^{Fuc}), 22.5, 26.3, 31.7 (α-C, β-C, γ-C), 66.7, 68.0, 69.8, 71.5 (2-C^{Fuc}, 3-C^{Fuc}, 4-C^{Fuc}, 5-C^{Fuc}), 75.4 (1-C^{Fuc}).

### Beispiel 21

Eine Lösung von 23, 24, 25 oder 26 (100-200 mg) in Methanol (5-10 ml) versetzt man mit Pd/C (10% Pd auf Aktivkohle, 100 mg) und hydriert 2-20 h unter Wasserstoffatmosphäre. Der Ansatz wird mit durch einen sterilen Spritzenfilter (0.2 mm, Schleicher & Schuell) filtriert und die Lösung i. Vak. eingeengt. Ausschlußchromatographie an Biogel® P2 mit Wasser als Eluent führt zu den Produkten mit hydrierter Doppelbindung in nahezu quantitativen Ausbeuten.

### Beispiel 22

Eine Mischung von C-allyl-fucopyranosid 8 (300 mg, 1.6 mmol), p-Iodanilin (525 mg, 2.4 mmol) und Natriumcarbonat (398 mg, 4.8 mmol) in Dimethylformamid (5 ml) unter Argonathmosphäre wird nach dreimaligem Entgasen mit Pd-Acetat (2 mol/%) versetzt. Man erhöht binnen 3 h die Temperatur auf 80°C, filtriert anschließend durch Kieselgur und entfernt die Lösungsmittel i. Vak.. Der Rückstand wird in wenig Wasser aufgenommen, durch einen sterilen Spritzenfilter (0.2 µm, Schleicher & Schuell) filtriert und zuletzt an RP18 mit Methanol/Wasser als Eluent gereinigt. Umfällen aus Wasser mit Aceton liefert 27 als amorphen Feststoff (236 mg, 0.85 mmol, 53 %).
¹H-NMR (300 MHz, D₂O): δ = 1.0 (d, 3 H, J_{6,5} = 6 Hz, 6-H^{Fuc}), 2.3 (m, 2 H, α-CH₂), 3.5-4.1 (5 H, 1-H^{Fuc}, 2-H^{Fuc}, 3-H^{Fuc}, 4-H^{Fuc}, 5-H^{Fuc}), 6.0 (dt, 1 H, β-H), 6.4 (d, 1 H, γ-H), 7.2 (m, 4 H, Ar).

### Beispiel 23

In Analogie zu Beispiel 17 werden mit dem C-allyl-fucopyranosid
a) o-Bromphenylessigsäure zu 28, m-Bromphenylessigsäure zu 28a,
b) m-Brombenzoesäureethylester zu 29,
c) o-Iodanilin zu 30,
d) 5-Brom-3-indolylessigsäure zu 31,
e) 4-(4-Bromphenyl)- 4-hydroxy-piperidin zu 32 umgesetzt.

### Beispiel 24

Eine Lösung von p-[Boc-N-Phenylalanin]-C-allyl-fucopyranosid (200 mg) in 5 ml DMF wird in rascher Folge mit Piperidin-4-Carbonsäure Ethylester (88 ml), HOBT (89 mg), HBTU (250 mg), DIPEA (115 ml) versetzt. Nach 3h werden die Lösungsmittel i. Vak. entfernt. DC [Dichlormethan/Methanol : 5/1]: R_{f} = 0.52. Der Rückstand wird in Methanol gelöst, dann mit 20 ml Wasser versetzt und schließlich mit Natronlauge bei pH 11.5-12 zu 33 verseift. DC [Dichlormethan/Methanol : 5/1]: R_{f} = 0.17

### Beispiel 25

Eine Lösung von o-Phenylessigsäure-C-allyl-fucopyranosid (100 mg) in 5 ml DMF wird in rascher Folge mit Piperidin-4-Carbonsäure Ethylester (60 µl), HBTU (171 mg), DIPEA (81µl) versetzt. Nach 4h werden die Lösungsmittel i. Vak. entfernt. DC [Dichlormethan/Methanol : 5/1]: R_{f} = 0.56. Der Rückstand wird in Methanol (5 ml) gelöst und dann mit Natronlauge (10 ml, 1N) zu 34 verseift.

### Beispiel 26

Ein Tetrapeptid mit RGDS-Sequenz wird mittels Fmoc-Festphasenstrategie an säurelabilen TCP-Harz ( K. Barlos, D. Gatos, J. Kallitsis, G. Papaphotiou, Y. Wenqing, W. Schäfer, Tetrahedron Lett. 1989, 30, 394.) aufgebaut (TCP-Ser(OtBu)-Asp(OtBu)-Gly-Arg(Pmc)-Fmoc).
Festphasenpeptidsynthese an TCP-Harz:
a) 20 % Piperidin in DMF
b) 3 Äquiv.temporär blockierte Aminosäure,
   HBTU/HOBT/DIPEA (jeweils 3 Äquiv.)

Zunächst erfolgt Deblockierung der N-terminalen Fmoc-Schutzgruppe mit 20% Piperidin in DMF. Das Harz (2.6 g, 1 mmol) mit dem partiell geschützten Tetrapeptid wird in 20 ml suspendiert und in schneller Folge mit p-[Boc-N-Phenylalanin]-C-allyl-fucopyranosid (541 mg), HOAT (163 mg), HATU (456 mg), DIPEA (209 µl) versetzt. Nach Waschen mit DMF wird das Harz für 3h mit Trifluoressigsäure (5% Wasser) behandelt. Die Lösungsmittel werden im Vakuum entfernt und durch Säulenchromatographie an RP18 mit Wasser/Methanol als Eluent 35 (344 mg) erhalten.
DC [Dichlormethan/Methanol : 2/1]: R_{f} = 0.6.- ¹H-NMR (300 MHz, D₂O): δ = 1.15 (d, 3 H, J_{6,5} = 6.4 Hz, 6-H^{Fuc}), 2.6 (m, 2 H, α-CH₂), 3.7-4.3 (6 H, 1-H^{Fuc}, 2-H^{Fuc}, 3-H^{Fuc}, 4-H^{Fuc}, 5-H^{Fuc}, α-H^{Phe}), 6.03 (m, 1 H, Hz , β-H), 6.57 (d, 1 H, J_{γ,β} = 15.8 Hz, γ-H), 7.25 (d, 2 H Ar), 7.43 (d, 2 H Ar); ¹³C-NMR (100.6 MHz, D₂O): δ = 18.0 (6-C^{Fuc}), 171.1, 173.3, 174.7, 175.3, 177.6, 178.5 (C=O). Anschließende Hydrierung von 35 (150 mg) für 3h in Methanol/AcOH (10 ml:10 ml) mit 100 mg Pd/C unter Wasserstoffathmosphäre führt nach Abfiltrieren und Einengen i. Vak zu 36 (143 mg) Ser-Asp-Gly-Arg-[C-FucPhe].
DC [Dichlormethan/Methanol : 2/1]: R_{f} = 0.59.- ¹H-NMR (300 MHz, D₂O): δ = 1.16 (d, 3 H, J_{6,5} = 6.2 Hz, 6-H^{Fuc}), 2.6 (m, 2 H, α-CH₂), 3.65-4.1 (6 H, 1-H^{Fuc}, 2-H^{Fuc}, 3-H^{Fuc}, 4-H^{Fuc}, 5-H^{Fuc}, α-H^{Phe}), 7.25 (m, 4 H Ar); ¹³C-NMR (100.6 MHz, D₂O): δ = 17.9 (6-C^{Fuc}), 171.4, 173.2, 174.5, 175.4, 177.4, 177.9 (C=O)

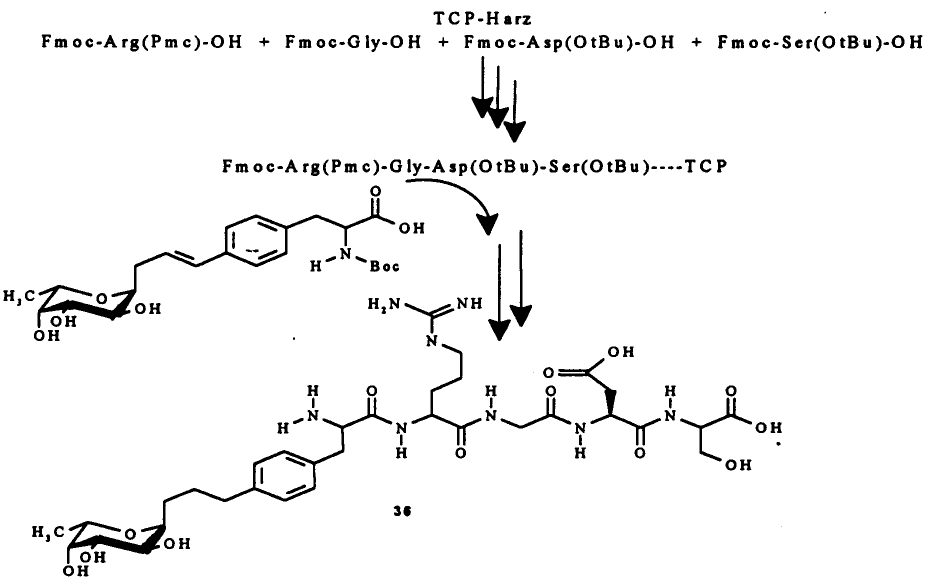

### Beispiel 27

Eine Mischung von C-allyl-fucopyranosid 8 (1.422 g, 7.55 mMol), (2R,3R)-4-Brom-1- carboxy-2,3-dihydroxycyclohexa-4,6-dien (2.660 g, 11.32 mMol, Janssen) und Natrium- hydrogencarbonat (1.898 g, 22.6 mMol) in Dimethylformamid (50 ml) wird unter Argonatmosphäre mit Pd(OAc)₂ (85 mg, 5 mol/%) versetzt und bei einer Temperatur von 60°C für 1.5 h gerührt. Man filtriert die Feststoffe ab, entfernt die Lösungsmittel i. Vak., nimmt den verbleibenden Feststoff in wenig Methanol auf und reinigt über eine kurze Säule an Kieselgel mit Dichlormethan/Methanol (1:3) als Eluent.
Mitteldruckchromatographie an Bakerground RP18 mit Wasser/Methanol (9:1→5:1 in 40 min, 5:1→1:9 in 17 min) als Eluent führt zu 37 als amorphem Feststoff (1.847 g, 71 %).
DC [Butanol/Essigsäure/Wasser : 3/1/1]: R_{f} = 0.66. - ¹H-NMR (300 MHz, D₂O): δ = 1.07 (d, 3 H, J_{6,5} = 6.21 Hz, 6-H^{Fuc}); 1.54-1.61, 2.59-2.64 (m, 6 H, α-CH₂, β-CH₂, γ-CH₂), 3.50-3.95 (2-H^{Fuc}, 3-H^{Fuc}, 4-H^{Fuc}, 5-H^{Fuc}, 1-H^{Fuc}), 6.70 (d, 1 H, J = 7 Hz, Ar), 7.31 (d, 1 H, J = 7 Hz, Ar); ¹³C-NMR (75.4 MHz, CDCl₃): δ = 16.47, 23.70, 25.94, 29.98, 67.47, 68.92, 70.66, 72.65, 76.38, 117.04, 120.3, 122.1, 134.73, 142.46, 149.90, 165.71, 176.46; FAB-MS: 341 (M-H)⁻.

## Patentansprüche

1. Verbindung der Formel I 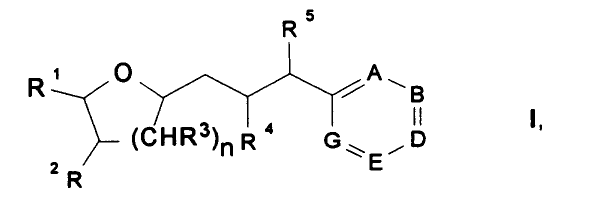 worin bedeuten
n 1 oder 2,
R¹ -H, -CH₂OH oder -CH₃,
R² und R³ unabhängig voneinander -H oder -OH,
R⁴ und R⁵ unabhängig voneinander -H, -OH, -Alkyl, -O-Alkyl, -S-Alkyl, -NH₂, -NH-Alkyl, -N(Alkyl)₂, -NH-Aryl, -N(Aryl)₂, -OSO₃H, -(CH₂)r-COOH, -(CH₂)ᵣ-COO-Alkyl, -(CH₂)ᵣCH(COO-Alkyl)₂, -(CH₂)ᵣCH(COOH)₂, -(CH₂)ᵣ-NH₂, wobei r eine ganze Zahl von null bis zehn bedeutet, oder gemeinsam eine Doppelbindung oder einen Epoxidring,
A, B, D, E und G CR⁶, CR⁷, CR⁸, CR⁹, CR¹⁰ oder Stickstoff mit der Maßgabe, daß jeweils nur eine der Variablen A, B, D, E oder G Stickstoff bedeutet, und
R⁶, R⁷, R⁸, R⁹ und R¹⁰ unabhängig voneinander -H, -OH, -Alkyl, -O-Alkyl, -NH₂, -NH-Alkyl, -N(Alkyl)₂, -NH-Aryl, -N(Aryl)₂, -F, -Cl, -Br, -I, -COO-Alkyl, -CO-NH₂, -COOH, -OSO₃H, 4-Hydroxy-piperidin-4-yl, -(CH₂)ₘ-COOH, -(CH₂)ₘCOO-Alkyl, -(CH2)ₘ-CH(COO-Alkyl)₂, -(CH₂)ₘ-CH(COOH)₂, wobei m eine ganze Zahl von null bis zehn bedeutet, -(CH₂)ₚ-NH₂, wobei p eine ganze Zahl von eins bis zehn bedeutet, eine Gruppe der Formel II 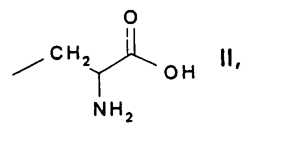 eine Gruppe der Formel III 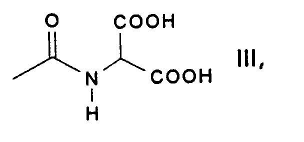 eine Gruppe der Formel IV 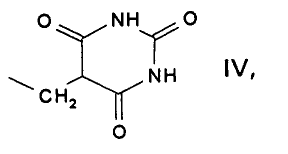 eine Gruppe der Formel V 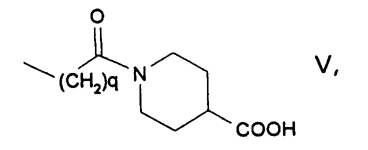 wobei q eine ganze Zahl von eins bis zehn bedeutet, oder eine Gruppe der Formel VI 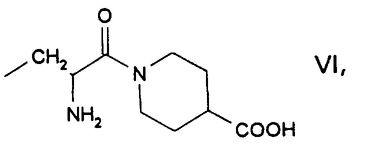 oder eine Gruppe der Formel VII 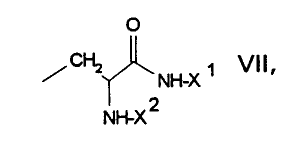 wobei X¹ und X² unabhängig voneinander H oder ein Oligopeptid darstellen oder
zwei der Variablen R⁶, R⁷, R⁸,R⁹ oder R¹⁰ bilden, sofern diese benachbart sind, gemeinsam einen mit einer Carboxymethylgruppe substituierten Imidazolring oder einen Kronenetherring, wobei die übrigen Variablen die genannte Bedeutung haben.

2. Verbindung nach Anspruch 1, dadurch gekennzeichnet, daß eine der Variablen A,B, G, E oder D C-COOH und alle übrigen dieser Variablen C-H bedeuten.

3. Verbindung nach Anspruch 1, dadurch gekennzeichnet, daß eine der Variablen A,B, G, E oder D C-CH₂-COOH und alle übrigen dieser Variablen C-H bedeuten.

4. Verbindung nach Anspruch 1, dadurch gekennzeichnet, daß eine der Variablen A,B, G, E oder D C-CH(COOH)₂ und alle übrigen dieser Variablen C-H bedeuten.

5. Verbindung nach Anspruch 1, dadurch gekennzeichnet, daß eine der Variablen A,B, G, E oder D C-NH₂ und alle übrigen dieser Variablen C-H bedeuten.

6. Verbindung nach Anspruch 1, dadurch gekennzeichnet, daß die Variablen A, D und G C-H, die Variable B Stickstoff und die Variable E C-CH₂-COOH bedeuten.

7. Verbindung nach Anspruch 1, dadurch gekennzeichnet, daß
A,B, G und E C-H und
D CR⁸ bedeuten.

8. Verbindung nach Anspruch 1 oder 7, dadurch gekennzeichnet, daß
R⁸ eine Gruppe der Formel II 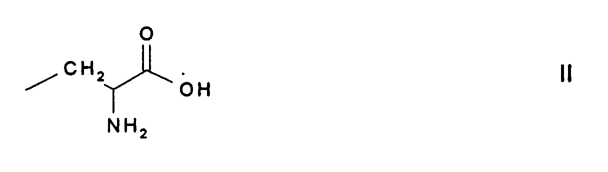
bedeutet.

9. Verbindung nach Anspruch 1 oder 7, dadurch gekennzeichnet, daß
R⁸ eine Gruppe der Formel III 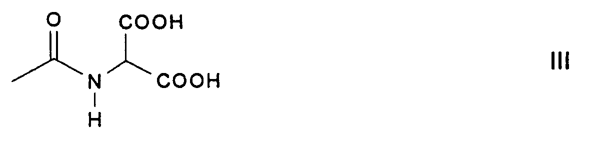
bedeutet.

10. Verbindung nach Anspruch 1 oder 7, dadurch gekennzeichnet, daß
R⁸ eine Gruppe der Formel IV 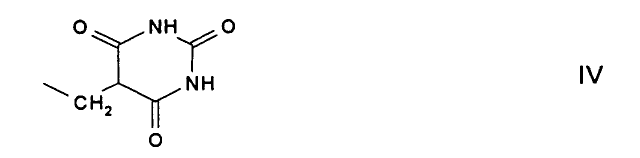
bedeutet.

11. Verbindung nach Anspruch 1 oder 7, dadurch gekennzeichnet, daß
R⁸ eine Gruppe der Formel V 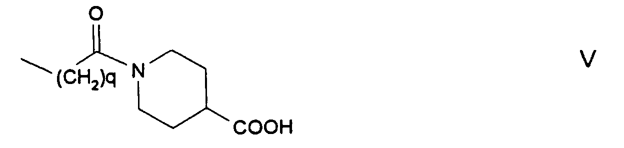 bedeutet, wobei q eine ganze Zahl von eins bis zehn darstellt.

12. Verbindung nach Anspruch 1 oder 7, dadurch gekennzeichnet, daß
R⁸ eine Gruppe der Formel VI 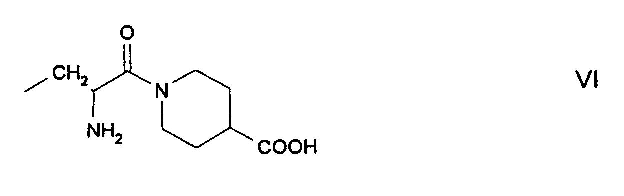 bedeutet.

13. Verbindung nach Anspruch 1 oder 7, dadurch gekennzeichnet, daß
R⁸ eine Gruppe der Formel VII 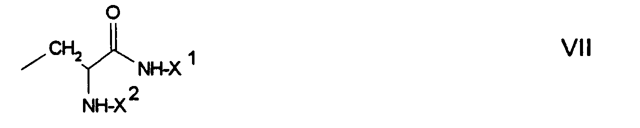 bedeutet, wobei X¹ und X² unabhängig voneinander H oder ein Oligopeptid darstellen.

14. Verbindung nach Anspruch 1, dadurch gekennzeichnet, daß eine der Variablen A,B, G, E oder D CR⁹ und alle übrigen dieser Variablen C-H bedeuten.

15. Verbindung nach Anspruch 1 oder 14, dadurch gekennzeichnet, daß R⁹ eine 4-Hydroxypiperidin-4-yl-Gruppe bedeutet.

16. Verbindung nach Anspruch 1, dadurch gekennzeichnet, daß die Variablen E und G gemeinsam einen substituierten Imidazolring bilden und die übrigen Variablen A, B und D C-H bedeuten.

17. Verbindung nach Anspruch 1, dadurch gekennzeichnet, daß die Varriablen A und B C-H, die Variablen G und E C-OH und die Variable D C-COOH bedeuten.

18. Verfahren zur Herstellung einer Verbindung gemäß einem der Ansprüche 1 bis 17, dadurch gekennzeichnet, daß man eine Verbindung der Formel VIII 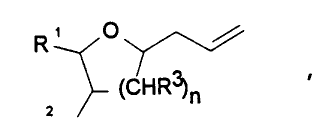 worin die Substituenten R¹, R² und R³, welche gegebebenfalls in geschützter Form vorliegen können, und die Variable n die in Anspruch 1 genannten Bedeutungen haben, unter der Wirkung eines Übergangsmetallkatalysators umsetzt mit einer Verbindung der Formel IX 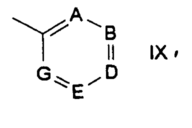 worin die Variablen A, B, D, E und G die in Anspruch 1 genannten Bedeutungen haben und X ein Halogenatom darstellt und wobei die Bindung zwischen G und E auch eine Einfachbindung sein kann, wonach man das Reaktionsprodukt gegebenenfalls derivatisiert und gegebenenfalls nach Abspaltung sämtlicher Schutzgruppen die Verbindung der Formel I gemäß einem der Ansprüche 1 bis 17 isoliert.

19. Verfahren nach Anspruch 18, dadurch gekennzeichnet, daß die Substituenten R¹, R² und R³ der Verbindung der Formel VIII in ungeschützter Form vorliegen.

20. Verfahren nach Anspruch 18 oder 19, dadurch gekennzeichnet, daß der Übergangsmetallkatalysator ein Palladiumkatalysator ist.

21. Arzneimittel enthaltend wenigstens eine Verbindung gemäß einem der Ansprüche 1 bis 17.

22. Verwendung einer Verbindung gemäß einem der Ansprüche 1 bis 17 für die Herstellung eines Arzneimittels zur Therapie oder Prophylaxe von Krankheiten, welche mit einer übermäßigen, durch Selektinrezeptoren vermittelten Zelladhäsion in dem von der Krankheit betroffenen Gewebe einhergehen.
